(19) Europäisches Patentamt
European Patent Office
Office européen des brevets

(11) Veröffentlichungsnummer : **0 144 366**
**B1**

(12) # EUROPÄISCHE PATENTSCHRIFT

(45) Veröffentlichungstag der Patentschrift : **29.07.87**

(21) Anmeldenummer : **84901990.6**

(22) Anmeldetag : **10.05.84**

(86) Internationale Anmeldenummer : **PCT/EP 84/00141**

(87) Internationale Veröffentlichungsnummer : **WO/8404520 (22.11.84 Gazette 84/27)**

(51) Int. Cl.[4] : **C 07 C 69/12**, C 07 C 69/24, C 07 C 69/527, C 07 C 43/115, C 07 C 43/162, C 11 B 9/00, A 23 L 1/226// C07C13/61, C07C35/37

(54) **GEMISCHE VON TRICYCLODECAN-DERIVATEN SOWIE DEREN HERSTELLUNG UND VERWENDUNG ALS RIECH- UND AROMASTOFF.**

(30) Priorität : **13.05.83 DE 3317476**

(43) Veröffentlichungstag der Anmeldung : **19.06.85 Patentblatt 85/25**

(45) Bekanntmachung des Hinweises auf die Patenterteilung : **29.07.87 Patentblatt 87/31**

(84) Benannte Vertragsstaaten : **CH FR GB LI NL**

(56) Entgegenhaltungen :
**EP-A- 0 039 232**
**DE-A- 2 642 519**
**DE-A- 3 208 203**
**FR-A- 2 334 343**
**Journal of the American Chemical Society, volume 85, April 1963 (Columbus, Ohio, US) L.A. Brown et al.: "The reaction of sodium borohydride with a nickel acetate in ethanol solution", pages 1004-1005. see the whole article cited in the application**
**Liebigs Annalen der Chemie, issue 11. November 1973 (Weinheim, DE) E. Klein et al.: "Reaktion von Cyclododecen mit Paraformaldehyd", page 1797, see the whole document cited in the application**

(73) Patentinhaber : **Dragoco Gerberding & Co. GmbH**
**Dragocostrasse 1**
**D-3450 Holzminden (DE)**

(72) Erfinder : **BRUNKE, Ernst-Joachim**
**Holbeinstrasse 6**
**D-3450 Holzminden (DE)**
Erfinder : **STRUWE, Hartmut**
**Am Feldberg 25**
**D-3470 Höxter 1 - Strahle (DE)**

(74) Vertreter : **Patentanwälte Müller-Boré, Deufel, Schön, Hertel, Lewald, Otto**
**Postfach 26 02 47 Isartorplatz 6**
**D-8000 München 26 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 144 366 B1**

Jouve, 18, rue St-Denis, 75001 Paris, France

**Beschreibung**

Das Dimere des Cyclopentadiens (1), Dicyclopentadien (2), ist ein wichtiges Ausgangsmaterial für die Produktion von Riechstoffen. Die durch Addition von Carbonsäuren (z. B. Essigsäure, Propionsäure) erhaltenen Ester 3b-c (Darstellung : Zeilanov et a., Chem. Abstracts, 68, 49319d) sind weltweit in großen Mengen verwendete Riechstoffe. Das Formiat 3a ist in dem UK-Patent 815, 232 (24.6.1959) und das Dimethylacrylat 3d in dem US-Patent 3 593 745 (10.8.1971) beschrieben worden. Weitere Riechstoffe aus Dicyclopentadien werden von Ohloff und Rodé-Gawal zusammenfassend beschrieben (in : H. Aebi, E. Baumgartner, H. P. Fiedler und G. Ohloff, « Kosmetika, Riechstoffe und Lebensmittelzusatzstoffe », G. Thieme Verlag, Stuttgart 1978, S. 55-57).

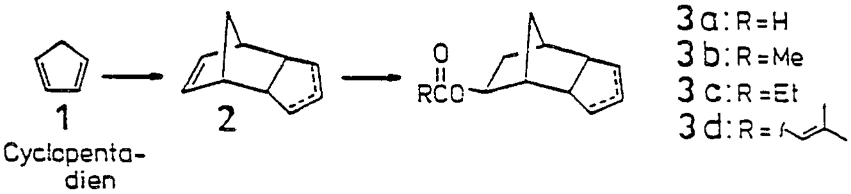


1 2

Cyclopentadien

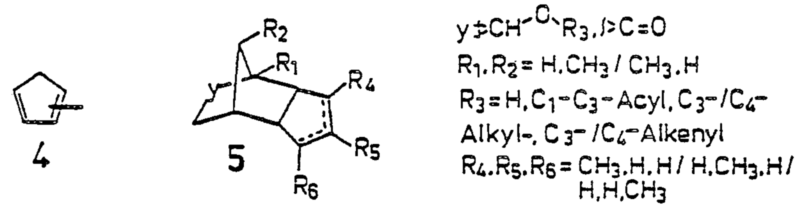


4 5

Über Produkte aus den Dimeren des Methylcyclopentadiens (4) ist wenig bekannt. In der Europ. Pat. Appl. 0 039 232 werden die aus den Dimeren des Methylcyclopentadiens (4) erhaltenen Dimethyl-Tricyclodecan-Derivate der allgemeinen Formel 5 als Riechstoffe beansprucht, wobei die gestrichelte Linie eine fakultative Doppelbindung bedeutet. Die Methylgruppe im Norbornan-Teil soll alternativ in den Resten $R_1$ oder $R_2$ und die Methylgruppe im Cyclopenten-Teil alternativ in den Resten $R_4$, $R_5$ oder $R_6$ enthalten sein. Die jeweils nicht substituierten Positionen $R_1$, $R_2$, $R_4$-$R_6$ sollen durch Wasserstoff besetzt sein. Der Molekülteil Y soll eine Carbonyl-, Hydroxyl-, Acetat-, Propionat-, bzw. eine $C_3$- oder $C_4$-Ethergruppe bedeuten. Die Produkte liegen als Gemische von Konstitutionsisomeren vor.

In der Deutschen Auslegeschrift 1 218 643 (6.6.1966) werden die Verbindungen der allgemeinen Formel 6 beansprucht, die Riechstoff-Eigenschaften besitzen sollen.

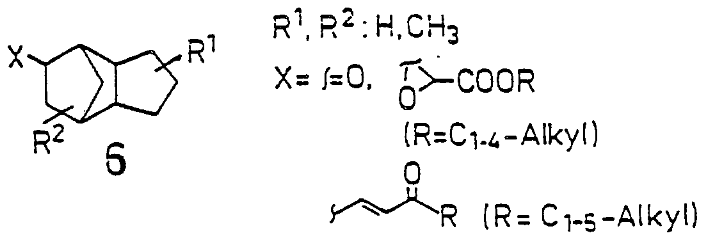


6

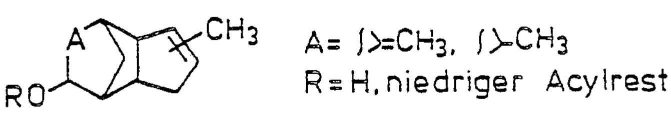


7

Die Deutsche Offenlegungsschrift 3 120 700 A1 (25.2.1982) beschreibt die Tricyclodecan-Derivate der allgemeinen Formel 7 und deren Verwendung als Riechstoffe.

Für die Darstellung der neuen Verbindungen der allgemeinen Formel A wurde von dimerem Methylcyclopentadien ausgegangen.

Methylcyclopentadien (Darstellung : Übersicht W. T. Ford, J. Org. Chem. 25, 3979 (1971)) liegt als

Gemisch der drei Doppelbindungsisomeren 4a, b, c vor. Nach S. McLean und P. Haynes (Tetrahedron 21, 2313 (1965)) liegen im Gleichgewicht 44,5 % des 1-Methyl-Isomeren 4a, 54,5 % des C-2-Isomeren 4b und nur ca. 1 % des C-5-Isomeren 4c vor.

Bei der Dimerisierung eines Gleichgewichtsgemisches von Methylcyclopentadien kann theoretisch eine Vielzahl von Produkten gebildet werden. Unter der Annahme, daß Produkte mit angulärer Methylgruppe sterisch ungünstig sind und nicht in nennenswerter Menge entstehen, und daß die von 4c (< 1 %) abzuleitenden Produkte vernachlässigt werden können, sollten aus Methylcyclopentadien bei Dimerisierung (Gaschromatogramm : Abb. 1) im wesentlichen die Konstitutionsisomeren 8a-h entstehen. Das $^1$H-NMR-Spektrum (Abb. 2) zeigt im Bereich olefinischer Protonen (5-6 ppm) Signalgruppen, deren Integral 2,2 Protonen entspricht. Die Signale bei 1.25 und 1.50 ppm können in Analogie zum Dicyclopentadien (Sadtler-NMR-Spektren-Sammlung Nr. 6494 M) den Protonen der $C_1$-Brücke zugeornet werden. Nur die Signale relativ geringer Intensität bei 1.23 und 1.35 ppm können den Brückenkopf-Methylgruppen von 8a-d zugeordnet werden. Dieser Befund und die Intensität der olefinischen Signale zeigen das überwiegende Vorliegen der Isomeren 8e-h (ca. 85 %), während 8a-d nur zu ca. 15 % enthalten sind.

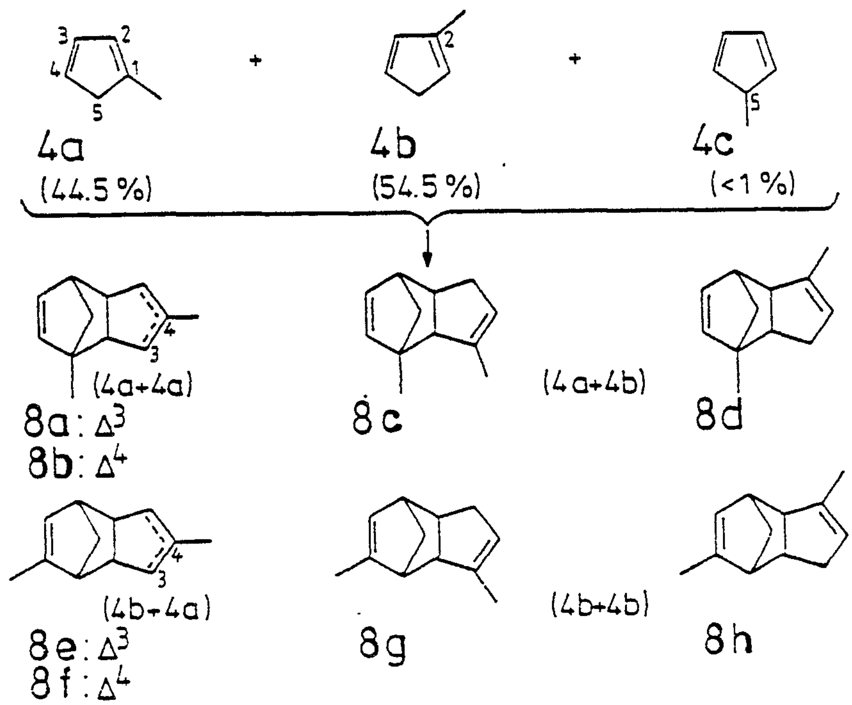

4a (44.5 %) 4b (54.5 %) 4c (<1 %)

(4a+4a) 8a: Δ3 8b: Δ4 8c (4a+4b) 8d

(4b+4a) 8e: Δ3 8f: Δ4 8g (4b+4b) 8h

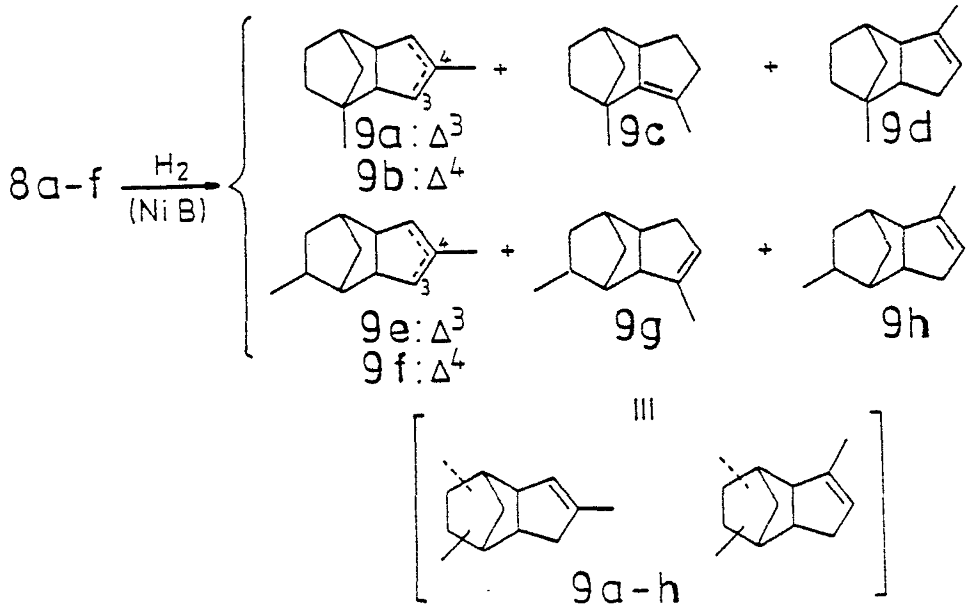


8a-f

9a: Δ3 9b: Δ4 9c 9d

9e: Δ3 9f: Δ4 9g 9h

9a-h

Die selektive Hydrierung des Norbornan-Teils der isomeren Dimethyldicyclopentadiene 8a-h gelang unter Modifikation einer von Ch. A. Brown für die selektive Hydrierung des Dicyclopentadiens (2) gegebenen Vorschrift (Ch. A. Brown, Chem. Commun. 1969, 952 ; H. C. Brown und Ch. A. Brown, J. Am. Chem. Soc., 85, 1004 (1963)). Unter Verwendung von Nickel-borid, das « in situ » erzeugt wurde, erfolgte die Hydrierung in Methanol, Ethanol oder einem anderen polaren, aprotischen Lösungsmittel bei Temperaturen von 20-80 °C, vorzugsweise bei 40-50 °C, und einem Wasserstoff-Druck von 30-70 bar, vorzugsweise 50-60 bar. Die dabei erhaltenen Mono-ene 9a-h liegen nach GC (Abb. 3) als Gemische aus drei Hauptisomeren (ca. 16, 24 und 37 %) sowie weiteren Nebenisomeren vor, bei denen es sich wie bei 8a-h um Konstitutionsisomere, hier aber auch Stereoisomere handeln kann. Alle diese Isomeren zeigen ein sehr ähnliches massenspektrometrisches Fragmentierungsmuster, das durch Öffnung des Norbornansystems unter Bildung eines Methylcyclopentadienyl-Radikalkations (m/z 80) und eines Methylcyclopentans erklärt werden kann. Im $^1$H-NMR-Spektrum (Abb. 4) charakterisieren Signale für olefinische Protonen bei $\delta$ = 5.1-5.3 ppm (1 H) und solche für olefinische Methylgruppen bei 1.7 ppm das Cyclopentensystem mit trisubstituierter Doppelbindung. Singuletts bei 1.25 und 1.15 ppm können Brückenkopf-Methylgruppen der Isomeren 9a-c und zwei überlagerte Dubletts bei 1.05 ppm den Methylgruppen im Norbornan-Teil der Isomeren 9d-f zugeordnet werden, die als Hauptisomere vorliegen.

Die neuen Verbindungen der allgemeinen Formel A wurden aus dem Gemisch der Olefine 9a-h durch Umsetzung mit Formaldehyd in Gegenwart von Säuren zugänglich. Der Mechanismus der Umsetzung von Olefinen mit Formaldehyd (« Prins-Reaktion ») bei unterschiedlichen Reaktionsbedingungen ist ausführlich untersucht worden (Übersicht in : E. Klein, F. Thömel, A. Roth und H. Struwe, Liebigs Ann. Chem. 1973, 1797). Die einzelnen Olefine des Gemischs 9a-h reagieren unterschiedlich mit Formaldehyd, und zwar die Isomeren 9a, b, e, f mit einer Methylgruppe an C-4 unter einfacher Addition zu den primären Alkoholen 10a-d (bzw. den Acetaten 12a-d) des Typs A und die Isomeren 9c, d, g, h mit einer Methylgruppe an C-3 oder C-5 unter zweifacher Addition zu dem tetracyclischen Ether des Typs B (11a-c). Bei Verwendung von p-Toluolsulfonsäure/Essigsäure/Acetanhydrid entsteht ein Gemisch der Acetate 12a-d und 11a-d und bei Verwendung von Ameisensäure (und alkalischer Aufarbeitung) ein Gemisch der Alkohole 10a-d und 11a-d. Die Produkte des Typs A und B werden unter den verschiedenen Reaktionsbedingungen in etwas unterschiedlichen Produktverhältnissen gebildet : bei Verwendung von Paraformaldehyd in Eisessig/Acetanhydrid mit katalytischen Mengen p-Toluolsulfonsäure (Beispiel 2/3 ; Gaschromatogramm des verseiften Reaktionsgemischs : Abb. 5) entstehen die tetracyclischen Ether in höherem Anteil als bei Verwendung von Paraformaldehyd in Ameisensäure (Beispiel 4 ; Gaschromatrogramm des Reaktionsgemisches : Abb. 6). Aus dem Reaktionsgemisch (PTS, Essigsäure, Acetanhydrid) wurden durch Destillation die Hauptisomeren 11d und 12d isoliert.

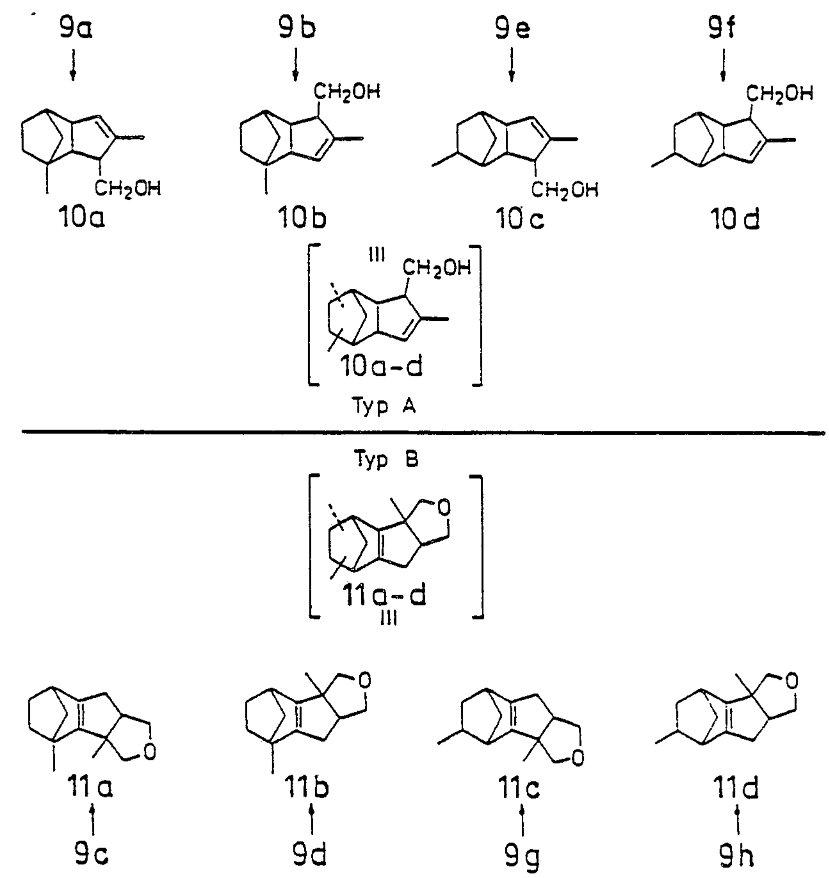

9a 9b 9e 9f

10a 10b 10c 10d

10a-d

11a-d

11a 11b 11c 11d

9c 9d 9g 9h

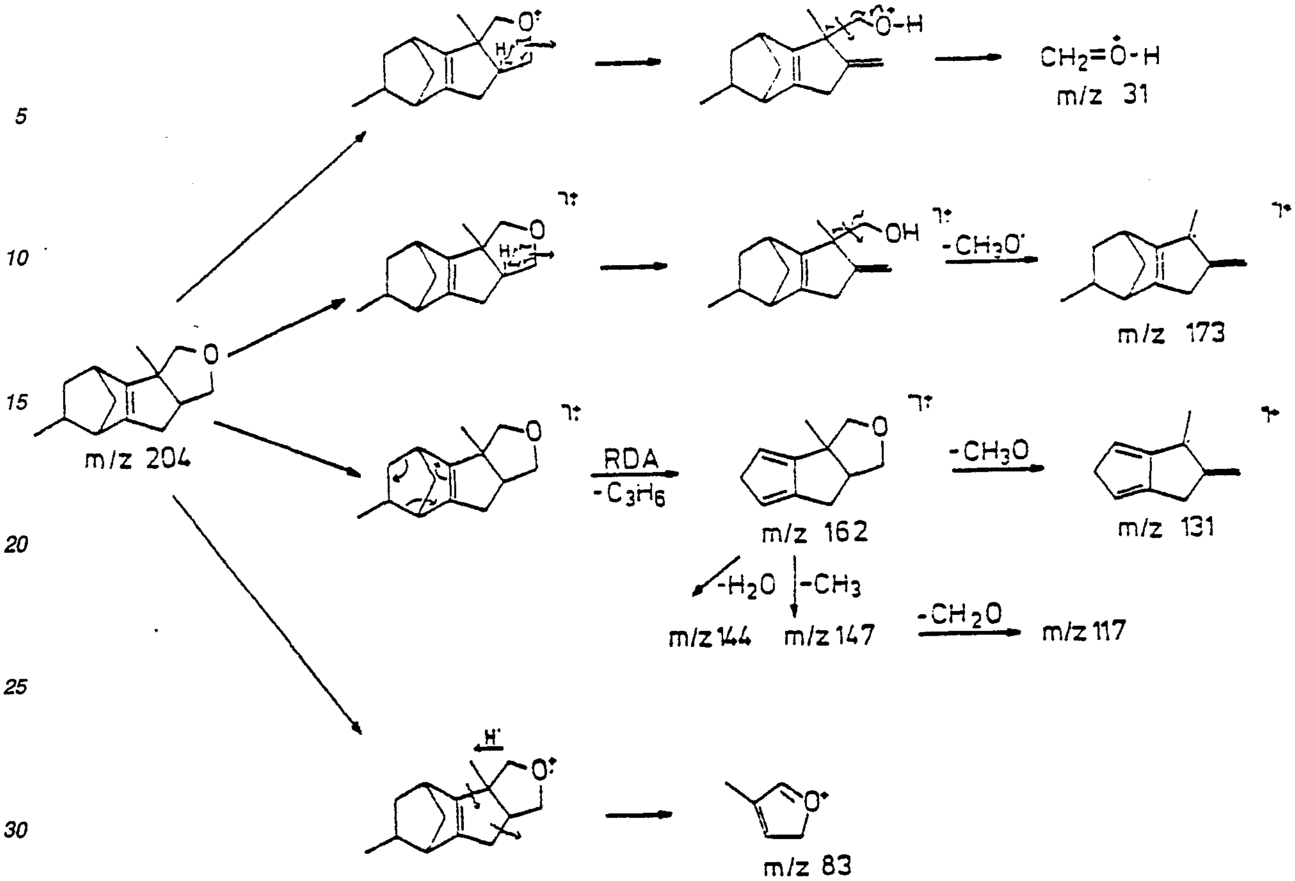


Massenspektrometrische Fragmentierung

Die Konstitution von 12d, dem Hauptisomeren des Typs A, ergibt sich aus dem $^1$H-NMR-Spektrum (Abb. 7) mit einem Dublett bei 0.99 (sek. Methyl), einem breiten Singulett bei 1.69 und einem Multiplett bei 5.41 ppm (olefinisches System), und zwei Dubletts bei 3.96 und 4.00 ppm (stereoisomere Acetoxymethylgruppen). Die massenspektrometrische Fragmentierung (Abb. 8) bestätigt die angegebene Struktur.

Die Konstitution von 11d, dem Hauptisomeren des Typs B, wird im $^1$H-NMR-Spektrum (Abb. 9) durch ein Dublett bei 0.84 ppm (Sek. Methyl), ein Singulett bei 1.18 (allylst. Methyl) sowie Multipletts bei 3.4-4.0 ppm (Tetrahydrofuransystem) und im $^{13}$C-NMR-Spektrum (Abb. 10) durch Singuletts bei 150 ppm (tetrasubstituierte Doppelbindung) charakterisiert. Die wesentlichen massenspektrometrischen Fragmentierungen von 11d lassen sich ohne Schwierigkeiten interpretieren und stehen mit der angegebenen Struktur in Einklang.

10a-d+11a-d

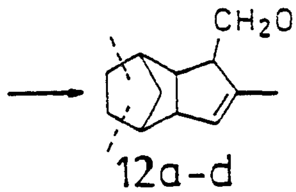

12a-d

+ 11a-d

10a-d+11a-d

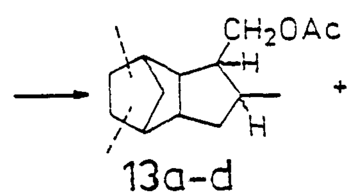

13a-d

+

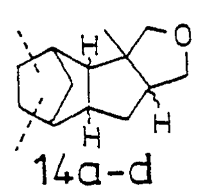

14a-d

Bei der Hydrierung der Produktgemische der Prins-Reaktion kann unter Normalbedingungen eine selektive Sättigung der trisubstituierten Doppelbindung (Verbindungen des Typs A) erzielt werden, so daß Gemische der Tricyclodecane 13a-d mit 11a-d entstehen. Verwendung von hohem Druck (100-200 bar) und erhöhter Temperatur ergibt auch die Sättigung der tetrasubstituierten Doppelbindung und führt zu Gemischen von 13a-d mit 14a-d.

Geruchsbeschreibungen

| | | 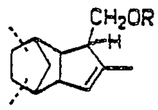 + 11a-d | | 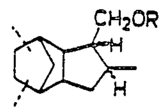 + 11a-d + 14a-d |
|---|---|---|---|---|
| R=H | 10a-d | frisch, holzig, blumig | 15a-d | holzig, frisch |
| R=Acetyl | 12a-d | holzig, krautig, süß | 13a-d | holzig, krautig, würzig |
| R=Propionyl | 16a-d | holzig, krautig | 32a-d | holzig, herb |
| R=Iso-butyryl | 17a-d | holzig-süß | 33a-d | holzig |
| R=Pivaloyl | 18a-d | weich-holzig (Kondensmilch) | 34a-d | süß, krautig-holzig |
| R=Tiglinyl | 19a-d | krautig, etwas süß | 34a-d | holzig-süß |
| R=Crotonyl | 20a-d | holzig-krautig (Liebstöckel) | 36a-d | holzig, würzig (Sellerie) |
| R=Methyl | 21a-d | holzig, etwas erdig | 37a-d | holzig, etwas Ambra |
| R=Ethyl | 22a-d | holzig, Ambra-Note | 38a-d | holzig, Ambra-Note |
| R=Allyl | 23a-d | holzig, fruchtig | 39a-d | fruchtig-holzig |
| R=Propyl | 24a-d | holzig | 40a-d | holzig |
| R=Methoxicarbonyl (Kohlensäureester) | 25a-d | holzig, süß-krautig | 41a-d | holzig, etwas medizinisch |
| R=Ethoxicarbonyl | 26a-d | holzig, süß-krautig | 42a-d | holzig, würzig |
| R=Formylmethyl | 27a-d | camphrig, frisch-holzig | 43a-d | camphrig-holzig |
| R=Dimethoxiethyl | 28a-d | frisch, holzig, fruchtig | 44a-d | frisch, holzig |
| R=Diethoxiethyl | 29a-d | frisch, holzig, fruchtig | 45a-d | frisch, holzig |
| R=Ethoximethyl | 30a-d | holzig, krautig, süß-herb | 46a-d | holzig, krautig |
| R=Methoximethyl | 31a-d | holzig, krautig, süß-herb | 47a-d | holzig, krautig |

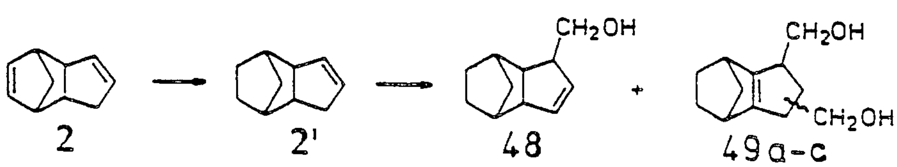

2 2' 48 49a-c

48 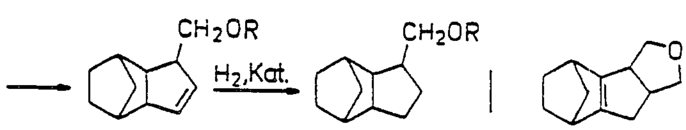


86

| | R | |
|---|---|---|
| 50 | Acetyl | 68 |
| 51 | Propionyl | 69 |
| 52 | Butyryl | 70 |
| 53 | Iso-butyryl | 71 |
| 54 | Pivaloyl | 72 |
| 55 | Tiglinyl | 73 |
| 56 | Crotonyl | 74 |
| 57 | Methyl | 75 |
| 58 | Ethyl | 76 |
| 59 | Allyl | 77 |
| 60 | Propyl | 78 |
| 61 | Methoxicarbonyl (Kohlensäureester) | 79 |
| 62 | Ethoxicarbonyl | 80 |
| 63 | Formylmethyl | 81 |
| 64 | Dimethoxiethyl | 82 |
| 65 | Diethoxiethyl | 83 |
| 66 | Ethoximethyl | 84 |
| 67 | Methoximethyl | 85 |
| | H | 87 |

Aus dem verseiften Produktgemisch der Prins-Reaktion, 10a-d + 11a-d, sowie dem Produktgemisch der selektiven Hydrierung 13a-d + 11a-d oder dem der vollständigen Hydrierung 13a-d + 14a-d wurden auf übliche Weise die entsprechenden Derivate der Alkoholkomponenten (Typ A), wie Ester, Ether, Acetate, Kohlensäureester und Oxoacetaldehyde dargestellt, die dann jeweils als Gemisch mit den tetracyclischen Ethern (Typ B) vorlagen.

Das dimere Cyclopentadien (2) kann nach der von Ch. A. Brown (Chem. Commun. 1969, 952), bzw. H. C. Brown und Ch. A. Brown (J. Am. Chem. Soc. 85, 1004 (1963)) gegebenen Vorschrift selektiv im Norbornan-Teil zu 2' hydriert werden. Prins-Reaktion von 2' mit Paraformaldehyd in Analogie zu den hier gegebenen Beispielen 2, 3 und 4 ergibt den ungesättigten Alkohol 48 und die Diole 49a-c, die sich z. T. zum tetracyclischen Ether 86 dehydratisieren lassen ; 48 kann durch fraktionierte Destillation isoliert werden. Durch Hydrierung unter Verwendung üblicher Katalysatoren wie Raney-Nickel, Platin oder Palladium/Aktivkohle wurde aus 48 der gesättigte Alkohol 68 erhalten. Aus 48 oder 68 wurden in Analogie zu den Beispielen 6-13 die zugehörigen Ester, Ether, Kohlensäureester und Acetale erhalten (Verbindungen 50-67, 69-85).

Die Gemische der Prins-Reaktion an 2' sowie die daraus in Analogie zu den Beispielen 5-13 hergestellten Derivate besitzen erdig-holzige, krautig-holzige und frische Geruchseigenschaften und können daher als Riech- und Aromastoff verwendet werden.

(Siehe Tabelle Seite 7 ff.)

| R | | 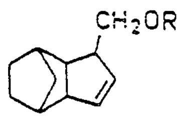 | | 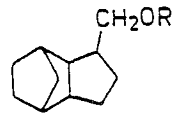 |
|---|---|---|---|---|
| Acetyl | 50 | süß, krautig holzig | 68 | holzig, würzig |
| Propionyl | 51 | holzig, krautig | 69 | holzig, herb |
| Butyryl | 52 | holzig | 70 | holzig |
| Iso-butyryl | 53 | holzig | 71 | holzig |
| Pivaloyl | 54 | weich, holzig, blumig | 72 | krautig-holzig |
| Tiglinyl | 55 | krautig | 73 | holzig-süß |
| Crotonyl | 56 | krautig, fettig | 74 | würzig (Sellerie) |
| Methyl | 57 | holzig, erdig | 75 | holzig, etwas Ambra |
| Ethyl | 58 | holzig, erdig | 76 | holzig |
| Allyl | 59 | holzig, fruchtig | 77 | fruchtig-holzig |
| Propyl | 60 | holzig | 78 | holzig |
| Methoxicarbonyl (Kohlensäureester) | 61 | holzig, frisch | 79 | holzig, etwas pilzig |
| Ethoxicarbonyl | 62 | holzig, frisch | 80 | holzig, frisch |
| Formylmethyl | 63 | camphrig, frisch, holzig | 81 | camphrig-holzig |
| Dimethoxiethyl | 64 | frisch, holzig | 82 | frisch, holzig |
| Diethoxiethyl | 65 | frisch, holzig | 83 | frisch, holzig, etwas fruchtig |
| Ethoximethyl | 66 | holzig, hell-krautig | 84 | holzig, krautig |
| Methoximethyl | 67 | holzig, hell-krautig | 85 | holzig, krautig |

Die in dieser Schrift beschriebenen Derivate des Dicyclopentadiens, die durch selektive Hydrierung der Doppelbindung im Norbornenteil und anschließende Prins-Reaktion an der Doppelbindung im Fünfring erhalten wurden, sind neu.

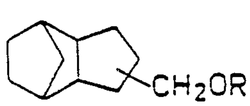

B

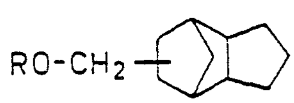

C

Sie unterscheiden sich durch ihre spektroskopischen Daten und ihre Geruchsnoten von den in der Dt. Offenleg. 26 54 268 genannten Stoffen, für die irrtümlich die gleiche Konstitutionsformel angegeben worden ist. Diese falsche Strukturzuordnung wurde bereits bei dem durch Hydroformylierung von Dicyclopentadien (Dt. Patent 934 889) gewonnenen Alkohol B (R = H) getroffen. In einer zugehörigen Veröffentlichung (H. P. Kaufmann und G. Steffan, Fette, Seifen, Anstrichm. 67, 784 (1965)) wurde die nunmehr als falsch erkannte Formel B dadurch abgeleitet, daß bei der Hydroformylierungsreaktion zunächst « die gespannte und daher energiereiche Doppelbindung im methylüberbrückten Sechsring bevorzugt hydriert wird », so daß die nachfolgende Einführung der Formylgruppe am Fünfring erfolgen muß. Die Annahme wurde seinerzeit nicht theoretisch begründet oder durch Versuche überprüft.

Der Abblauf der Hydroformylierungsreaktion (auch « Oxosynthese » genannt) ist seit längerem bekannt (Übersicht in : Advanced Organic Chemistry, McGraw Hill, New York 1977, S. 737 + 738). Bei der

Verwendung von Dicobaltoctacarbonyl als Katalysator bildet sich in Gegenwart von Wasserstoff und Kohlenmonoxid das Tetracarbonylhydrocobalt und wird an die Doppelbindung addiert ; dann folgt eine Umlagerung und Reduktion der C-Co-Bindung. Im Fall des Dicyclopentadiens sollte unter kinetischer Kontrolle eine Mono-hydroformylierung unter Anlagerung des Katalysators an die aktivere Doppelbindung im Norbornanteil erfolgen, so daß folglich auch die Formylgruppe am Norbornanteil eingeführt wird. Es wurde gezeigt, daß die Bildung des jeweiligen Alkohols ein zweiter, anschließender Rekationsschritt ist, wobei Tetracarbonylhydrocobalt als reduzierendes Agens wirkt (Aldridge und Jonassen, J. Am. Chem. Soc. 85, 886 (1963)). Im Fall des Dicyclopentadiens war daher mit der Bildung des am Norbornanteil substituierten Produkts C (R = H) zu rechnen. Eine eingehende Untersuchung hat inzwischen gezeigt, daß bei der Hydroformylierung des Dicyclopentadiens unter Verwendung von Rhodiumkatalysatoren bei milden Bedingungen der Einbau der Formylgruppe am Norbornanteil unter vollständiger Erhaltung der Fünfring-Doppelbindung erfolgte ; anschließende Hydrierung führte zu dem gesättigten Alkohol C (R = H) (Y. Fujikura, Y. Inamoto, N. Takaishi und H. Ideda, Synthetic Comm. 6, 199 (1976)).

Die Nichtidentität der von uns beanspruchten Verbindungen 48-85, die auf eindeutige Weise dargestellt worden sind, mit den in der Dt. Offenleg. 26 54 268 beschriebenen Verbindungen, denen richtigerweise die allgemeine Formel C zuordnen ist, ergibt sich zweifelsfrei aus den $^{13}C$-NMR-Spektren. Ein exemplarischer Vergleich ist anhand der Acetate 68 und C (R = Ac) möglich (Fig. 12, 13).

Die Gemische der neuen Verbindungen der allgemeinen Formel A zeichnen sich durch holzige, frische oder süß krautige Geruchsnoten aus und lassen sich vorteilhaft als Riechstoffe verwenden und in unterschiedlichen Parfümölen einarbeiten.

Die nachfolgenden Beispiele sollen die Erfindung erläutern, ohne die Anwendungsmöglichkeiten einzuschränken.

## Beispiel 1

Herstellung der Dimethyl-tricyclo [5.2.1.$0^{2,6}$] decene 9a-h

Zu einer Mischung von 139.5 g (0.56 mol) $Ni(Ac)_2 \cdot 4H_2O$ und 1.9 l Ethanol wurden unter Stickstoff-Atmosphäre 150 ml einer ethanolischen 1 m $NaBH_4$-Lösung unter Rühren zugetropft (15 min.) Hierbei fällt Nickelborid als feinkörniger, schwarzer Niederschlag aus. Nach Zugabe von 1.44 kg (9 mol) des dimeren Methylcyclopentadiens wurde die Stickstoff-Atmosphäre abgesaugt. Die Hydrierung erfolgte in einer Wasserstoff-Atmosphäre innerhalb von 16 Stdn. bei einem Druck von 50-60 bar und einer Temperatur von 40-50 °C. Nach Filtrieren, Einengen und Destillieren lagen 2.57 kg (88 %) 9a-h als farbloses Öl vor ; Kp (2.6 mbar) = 76-82 °C ; Dichte : $d_{4°}^{20°}$ = 0.941 7 ; Brechung $n_D^{20°}$ = 1.498 9. GC : Abb. 3, $^1H$-NMR : Abb. 4

$C_{12}H_{18}$(162.4)

## Beispiel 2

Prins-Reaktion an 9a-h (mit p-Toluolsulfonsäure)

Eine Lösung von 1 296 g (8 mol) 9a-h in 1 240 g Essigsäure (wasserfrei) wurde mit 74.4 g p-Toluolsulfonsäure und 457.6 (15.3 mol) Paraformaldehyd unter Rühren versetzt, und langsam auf 90 °C erhitzt. Nach 1 Stde. Rühren bei 90 °C ließ man 137.6 g Acetanhydrid innerhalb von 30 min zulaufen. Das Reaktionsgemisch wurde 6 Stdn. bei 90 °C gerührt. Nach Abkühlung wurde mit 1.8 l Wasser versetzt und mit Petroläther extrahiert. Die aufgearbeiteten organischen Phasen wurden eingeengt und über eine 20 cm-Vigreux-Kolonne destilliert. Man erhielt 1 250 g Rohprodukt ; Kp (1.0 mbar) = 75-165 °C. Durch Fraktionierung des Rohprodukts wurden 875 g (47 %) 12a-d + 11a-d als farbloses Öl erhalten ; $n_D^{20°}$ = 1.500 1, $d_{4°}^{20°}$ = 1.025 4.

Durch Destillation an einer Drehbandkolonne wurden aus dem Produktgemisch 12a-d + 11a-d als Hauptprodukte 12d und 11d isoliert.

5-Acetoxy-4,9-dimethyl-tricyclo [5.2.1.$0^{2,6}$] decen-3 (12d)

farbloses Öl ; $N_D^{20°}$ = 1.493 2, $d_{4°}^{20°}$ = 1.026 5. $^1H$-NMR (60 MHz, $CCl_4$) : δ = 0.99, d, J = 6.5 Hz (9—$CH_3$), 1.69, br · s (4—$CH_3$), 1.96, s (—$CH_2$—O—CO—$\underline{CH_3}$), 3.96 und 4.00, 2d, 2H (—$CH_2$—O—$COCH_3$), 5.41 ppm, m (3—H). IR (Film) : 1 740 $cm^{-1}$ (Ester). MS : m/z (%) = 174 (24, $M^+$-60), 159 (18), 132 (12), 131 (25), 119 (15), 118 (10), 117 (15), 105 (22), 95 (15), 94 (85), 93 (21), 92 (64), 91 (43), 81 (100), 80 (14), 79 (24), 77 (18), 43 (52), 41 (13).

$C_{15}H_{22}O_2$ (234.34)

4,5-Tetrahydrofurano-5,9-dimethyl-tricyclo [5.2.1.$0^{2,6}$] decen-2 (11d)

farbloses Öl ; $n_D^{20°}$ = 1.506 3, $d_{4°}^{20°}$ = 1.023 2. $^1H$-NMR (60 MHz, $CCl_4$ ; Abb. 9) : 0.84, d, J = 6.5 Hz

(9—$CH_3$), 1.18, s (5—$CH_3$), 3.4-4.0 ppm, m (—$CH_2$—O—$CH_2$) · $^{13}$C-NMR (Abb. 10) : 20.71 (q), 23.86 (q), 33.65 (t), 34.22 (d), 34.31 (t), 41.59 (d), 46.26 (d), 55.04 (d), 55.52 (s), 55.61 (t), 75.95 (t), 79.01 (t), 149.20 (s), 151.50 ppm (s). IR : 1 070, 1 065, 1 040, 1 005 $cm^{-1}$ (Ether). MS : m/z (%) = 204 (11, $M^+$), 162 (48), 147 (22), 132 (15) 131 (100), 130 (12), 129 (13), 117 (62), 115 (15), 91 (18), 39 (9).

$C_{14}H_{20}O$ (204.31)

Beispiel 3

Verseifung von 12a-d + 11a-d

Eine Lösung von 117 g 12a-d + 11a-d (Gemisch aus Beispiel 2) und 26 g NaOH in 350 ml Methanol und 9 ml Wasser wurde 1.5 Stdn. zum Sieden erhitzt. Nach Abdestillieren des Lösungsmittels wurden 150 ml Wasser zugegeben. Man extrahierte mit Benzin, arbeitete auf, destillierte das Rohprodukt über eine 20 cm-Vigreux-Kolonne und erhielt 90.2 g (94 %) 10a-d + 11a-d ; Kp (1.3 mbar) = 98 °C, $n_D^{20°}$ = 1.511 7, $d_{4°}^{20°}$ = 1.023 3. Gaschromatogramm Abb. 5.

WG 11, 50 m, 150-220 °C, 4 °C/min. Prins-Reaktionsprodukte (PTS, HOAc, $Ac_2O$)

Durch fraktionierte Destillation über eine Drehbandkolonne wurde das Isomere 10d isoliert.

5-Hydroxymethyl-4,9-dimethyl-tricyclo [5.2.1.$0^{2,6}$] decen-3 (10d)

farbloses Öl ; $^1$H-NMR ($CCl_4$) : 0.97, d, J = 7 Hz (9—$CH_3$), 172, br.s (4—$CH_3$), 3.5, s (—$CH_2$—OH), 5.33 ppm (3—H). IR : 355 0 $cm^{-1}$ (OH). MS : m/z (%) = 192 (15, $M^+$), 177 (2), 174 (5), 161 (42), 131 (6), 119 (11), 110 (17), 105 (32), 95 (30), 91 (39), 81 (100).

$C_{13}H_{20}O$ (192.30)

Beispiel 4

Prins-Reaktion an 9a-h (in Ameisensäure)

Man suspendierte 22.5 g (0.75 mol) Paraformaldehyd in 125 ml wasserfreier Ameisensäure und rührte solange bei Siedetemperatur, bis eine klare Lösung entstanden war (ca. 5 min). Bei ca. 30 °C ließ man 81 g (0.5 mol) Olefingemisch 9a-h zulaufen (leicht exotherme Reaktion). Die Reaktionsmischung wurde 20 Stdn. bei 38 °C gerührt, nach Abkühlung mit 300 ml Wasser versetzt und mit Benzin extrahiert. Die vereinigten organischen Phasen wurden 30 min mit Natronlauge (20 %ig) gerührt, danach neutralgewaschen und eingeengt. Destillation über eine 20 cm-Vigreux-Kolonne ergab 57.0 g (60 %) 10a-d + 11a-d als farbloses Öl ; Kp. (2 mbar) = 85-115 °C, $n_D^{20°}$ = 1.513 0, $d_{4°}^{20°}$ = 1.027 7. Gaschromatogramm : Abb. 6. WG 11, 50 m, 150-220 °C, 4 °C/min, Prins-Reaktionsprodukte (HCOOH).

Beispiel 5

Hydrierung von 11a-d + 12a-d

a) Selektive Hydrierung von 12a-d

Eine Lösung von 152 g 11a-d + 12a-d (Produktgemisch aus Beispiel 2) in 400 ml wasserfreier Essigsäure wurde mit 500 mg Platin-(IV)-oxid versetzt und in einer Wasserstoff-Atmosphäre bei Normalbedingungen geschüttelt, bis die Wasserstoff-Aufnahme beendet war. Nach Entfernen des Wasserstoffs wurde filtriert, eingeengt und über eine 20 cm-Vigreux-Kolonne destilliert. Man erhielt 129 g (85 %) 11a-d + 13a-d als farbloses Öl ; Kp (1 mbar) = 96-115 °C, $n_D^{20°}$ = 1.495 3, $D_{4°}^{20°}$ = 1,918 6. GC/MS : $t_r$ (%) = 30.3 (34, 11d), 34.07 (5, M = 206), 34.7 (3, M = 206), 34.9 (6, M = 236), 35.2 (3, M = 204), 36.5 (12, M = 236), 36.8 (22, M = 236), 37.1 (2.5, M = 236).

b) Vollständige Hydrierung

Eine Lösung von 152 g 11a-d + 12a-d (Produktgemisch aus Beispiel 2) in 300 ml Methanol wurde mit 3 g Raney-Nickel versetzt und in einer Wasserstoff-Atmosphäre bei 150 at/100 °C gerührt, bis die Wasserstoffaufnhame beendet war. Nach Entfernen des Wasserstoffs wurde filtriert, eingeengt und über eine 20 cm-Vigreux-Kolonne destilliert. Man erhielt 121 g (82 %) 13a-d + 14a-d als farbloses Öl.

5-Acetoxymethyl-4,9-dimethyl-tricyclo [5.2.1.$0^{2,6}$] decan (13d)

MS (Typ A) : m/z (%) = 176 (10, $M^+$-60), 161 (18), 147 (16), 134 (100), 133 (83), 121 (29), 119 (34), 107 (29), 106 (27), 105 (34), 93 (71), 79 (68).

$C_{15}H_{24}O_2$ (236.35).

4,5-Tetrahydrofuronao-5,9-dimethyl-tricyclo [5.2.1.0$^{2,6}$] decan (14d)

MS (Typ B) : m/z (%) = 206 (1, $M^+$), 176 (16), 161 (14), 147 (20), 134 (15), 121 (12), 120 (12), 107 (21), 93 (34), 81 (84), 80 (100).
$C_{14}H_{22}O$ (206.32).

## Beispiel 6

Verseifung von 13a-d + 11a-d

In Analogie zu Beispiel 3 wurde ein Gemisch von 15a-d + 11a-d erhalten ; $n_D^{20°}$ = 1.506 1, $d_{4°}^{20°}$ = 1.015 1.

5-Hydroxymethyl-4,9-dimethyl-tricyclo [5.2.1.0$^{2,6}$] decan (15d)

MS : m/z (%) = 194 (6, $M^+$), 176 (12), 163 (64), 147 (6), 121 (32), 107 (41), 105 (18), 94 (24), 93 (32), 81 (100), 80 (84), 79 (46), 67 (31), 55 (27).
$C_{13}H_{22}O$ (194.30)

## Beispiel 7

Veresterung von 10a-d + 11a-d, 13a-d + 11a-d, 13a-d + 14a-d

Eine Mischung von 77 g (0.4 mol, bezogen auf 10a-d bzw. 15a-d) des Produktgemisches nach Beispiel 3 (bzw. nach Beispiel 6) und 0.8 mol des jeweiligen Säureanhydrids wurden bei 60-80 °C portionsweise mit 19.5 g Natriumkarbonat unter Rühren versetzt. Nach 6 Stdn. Rühren bei 80-85 °C und Abkühlen gab man langsam 200 ml Wasser zu, extrahierte mit Benzin, arbeitete auf und destillierte über eine 20 cm-Vigreux-Kolonne. Es wurden die jeweiligen Ester in Ausbeuten von 75-85 % erhalten.

| | | $n_D^{20°}$ | $d_{4°}^{20°}$ |
|---|---|---|---|
| Acetate | 12a-d (+ 11a-d) | 1.5001 | 1.0254 |
| Propionate | 16a-d (+ 11a-d) | 1.4981 | 1.0178 |
| Isobutyrate | 17a-d (+ 11a-d) | 1.4960 | 1.0113 |
| Acetate | 13a-d (+ 11a-d) | 1.4953 | 1.0186 |
| Crotonate | 20a-d (+ 11a-d) | 1.5021 | 1.0233 |

## Beispiel 8

Darstellung der Methyl- oder Ethylether 21a-d + 11a-d, 22a-d + 11a-d

Zu einer Lösung von 77 g (0.4 mol, bezogen auf 10a-d, bzw. 15a-d) des Produktgemisches nach Beispiel 3 (bzw. Beispiel 5) und 4 g « Aliquat 336 » in 240 ml Benzin wurden bei 20-50 °C innerhalb von 1.5 Stdn. 82 g Natronlauge (50 %ig) zugetropft. Nach 1 Stde. Rühren bei 45 °C ließ man 103 g (87 ml, 0.67 mol) Diethylsulfat bzw. 84 g (63 ml, 0.7 mol) Dimethylsulfat zulaufen und rührte ca. 20 Stdn. bei 45 °C. Extraktion mit Benzin, Aufarbeiten und Destillation über eine 35 cm-Vigreux-Kolonne ergaben
66 g (60 %) 22a-d + 11a-d, Kp. (1.3 mbar) = 93-96 °C,
$n_D^{20°}$ = 1.498 4, $d_{4°}^{20°}$ = 0.991 6.
bzw.
61.5g (58 %) 21a-d + 11a-d, Kp. (1.8 mbar) = 88-91 °C.
$n_D^{20°}$ = 1.501 9, $d_{4°}^{20°}$ = 1.002 0.

5-Ethoxi-4,9-dimethyl-tricyclo [5.2.1.0$^{2,6}$] decen-3 (22d)

MS : m/z (%) = 220, (10, $M^+$), 161 (40), 119 (20), 118 (32), 105 (25), 95 (11), 94 (14), 93 (21), 92 (10), 91 (16), 81 (100), 79 (21), 77 (14), 55 (14).
$C_{15}H_{24}O$ (220.35)

5-Methoxi-4,9-dimethyl-tricyclo [5.2.1.0$^{2,6}$] decen (21d)

MS : m/z (%) = 206 (17, $M^+$), 161 (74), 159 (18), 123 (17), 119 (27), 118 (28), 109 (16), 105 (40), 93 (30), 91 (32), 81 (100), 79 (20), 77 (19), 45 (36).

$C_{14}H_{22}O$ (206.33)

Beispiel 9

Darstellung der Allylether 23a-d + 11a-d

Zu einer Lösung von 43 g (ca. 0.23 mol) des Produktgemisches nach Beispiel 3, bzw. Beispiel 5 in 67 ml Toluol wurden 13.5 g gepulvertes Natriumhydroxid und 3.4 g Aliquat 336 gegeben. Unter Rühren ließ man bei ca. 40 °C 21 g Allylchlorid zutropfen. Nach 12 Stdn. Rühren bei 40-45 °C gab man 100 ml Wasser zu, extrahierte mit Benzin und arbeitete auf. Destillation über eine 20 cm-Vigreux-Kolonne ergab ca. 55 g Produktgemisch 23a-d + 11a-d ; Kp. (15 mbar) = 88-90 °C.

$n_D^{20°}$ = 1.503 1, $d_{4°}^{20°}$ = 0.998 1.

23d : $C_{16}H_{24}O$ (232.27, bestätigt durch MS).

Beispiel 10

Darstellung der Pivalinate 18a-d + 11a-d

Zu einer Lösung von 67.2 g (ca. 0.35 mol) des Produktgemisches nach Beispiel 3 in 250 ml trockenem Pyridin wurden 84.3 g Pivalinsäurechlorid getropft (ca. 30 Min). Nach 2 Stdn. Rühren bei ca. 50 °C wurde aufgearbeitet. Destillation über eine 20 cm-Vigreux-Kolonne ergab 76 g Estergemisch 18a-d + 11a-d, Kp. (1.5 mbar) = 118-122 °C, $n_D^{20°}$ = 1.494 2, $d_{4°}^{20°}$ = 1.002 6.

18d : $C_{18}H_{28}O_2$ (276.42, bestätigt durch MS).

Beispiel 11

Darstellung der Kohlensäureester

Einer Lösung von 67.5 g des Produktgemisches nach Beispiel 3 in 36 g Pyridin und 276 ml trockenem Toluol wurden bei ca. 0 °C unter Rühren 49.5 g Chlorameisensäureethylester zugetropft. Nach 10 Stdn. Rühren wurden 300 ml Wasser zugegeben. Aufarbeitung und Destillation ergab 71 g Produktgemisch 16a-d + 11a-d ;

$n_D^{20°}$ = 1.496 3, $d_{4°}^{20°}$ = 1.037 6.

26d : $C_{16}H_{24}O_3$ (264.36) (Struktur bestätigt durch MS).

Methylester 25a-d + 11a-d : $n_D^{20°}$ = 1.499 0, $d_{4°}^{20°}$ = 1.046 2.

25d : $C_{15}H_{22}O_3$ (250.34) (Struktur bestätigt durch MS).

Beispiel 12

Darstellung der Formaldehydacetale 30a-d (+ 11a-d)

67.5 g des Produktgemisches nach Beispiel 3 und 187.5 g Formaldehyddiethylacetal wurden mit 3 g KSF-Katalysator 4 Stdn. gerührt. Anschließend wurde Ethylalkohol und überschüssiges Formaldehyddiethylacetal über eine 50 cm-Glasfüllkörperkolonne abdestilliert. Destillation des Rohprodukts ergab 65 g des Formaldehydethylacetals 30a-d (+ 11a-d) ; $n_D^{20°}$ = 1.504 3, $d_{4°}^{20°}$ = 1.018 1.

30d : $C_{16}H_{26}O_2$ (250.38, bestätigt durch MS).

Beispiel 13

Darstellung der Dimethoxi-ethyl-Derivate 28a-d (+ 11a-d) und der Oxoacetaldehyde 17a-d (+ 11a-d)

77 g des Produktgemisches nach Beispiel 3 wurden mit 24 g NaOH (fest) und 4 g Aliquat 336 vermischt und bei 140 °C mit 49 g (0.4 mol) Chloracetaldehyddimethylacetal versetzt. Nach 36 Stdn. Rühren bei Siedetemperatur wurde aufgearbeitet.

Dimethylacetat 28a-d (+ 11a-d) : $n_D^{20°}$ = 1.501 5, $d_{4°}^{20°}$ = 1.013 9

28d : $C_{17}H_{28}O_3$ (280.41)

56 g des Produktgemisches nach Beispiel 13 wurden mit 200 g Essigsäure 70 %ig vermischt und 1 Stde. bei 95 °C nachgerührt. Anschließend mit Wasser ausgedünnt, mit Benzin extrahiert und wie üblich aufgearbeitet.

Oxoacetaldehyde 27a-d (+ 11a-d) : $C_{15}H_{22}O_2$ (234.34).

Beispiel 14

Prins-Reaktion an Tricyclo [5.2.1.$0^{2,6}$] decen-4

In Analogie zu Beispiel 2 wurden 1 340 g (10 mol) Tricyclo [5.2.1.$0^{2,6}$] decen-4 in 1 400 g Essigsäure mit 74.4 g p-Toluolsulfonsäure und 458 g (15 mol) Paraformaldehyd umgesetzt und analog Beispiel 3 verseift. Das Rohprodukt wurde über eine 40 cm-Vigreux-Kolonne destilliert und ergab 720 g 48 und 240 g 49a-c als fablose Öle.

5-Hydroxymethyl-tricyclo [5.2.1.$0^{2,6}$] decen-3 (48)

GC (50 m FFAP, 230 °C) : $t_r$ = 37.4 min. $^1$H-NMR (60 MHz, $CCl_4$) : δ = 1.25 und 1.45, 2 br.s ($CH_2$), 2.1-2.4, 2.4-3.2, m (C—H), 3.36, d, J = 7 Hz (—$CH_2$—OH), 5.6 ppm (olefin. H). IR : 3 320 $cm^{-1}$ (OH). MS : m/z (%) = 164 (10, $M^+$), 146 (3), 133 (84), 117 (4), 115 (4), 105 (13), 96 (15), 95 (14), 91 (85), 79 (25), 77 (24), 67 (100).
$C_{11}H_{16}O$ (164.4)

3(4), 5-Di-hydroxymethyl-tricyclo [5.2.1.$0^{2,6}$] decane 49a-c

$^1$H-NMR (60 MHz, $CCl_4$) : δ = 3.2-4.2 ppm, m (—$CH_2$—OH). IR : 3 400 $cm^{-1}$ (OH). GC (50 m, FFAP, 230 °C) : $t_r$ (min) = 32.9 (10 %, 49a), 60.2 min (72 %, 49b), 60.5 (6 %, 49c). MS (49b, c) : m/z (%) = 194,6 ($M^+$), 176 (24), 162 (83), 149 (100), 131 (25), 117 (21), 105 (57), 91 (84), 82 (60), 79 (90), 66 (36), 55 (21), 41 (51).
$C_{12}H_{18}O_2$ (194.3).

## Beispiel 15

Spektroskopische Daten

5-Acetoxymethyl-tricyclo [5.2.1.$0^{2,6}$] decen-3 (50) (Darstellung aus 48 analog Beispiel 7)

GC (50 m, FFAP, 230 °C) : $t_r$ = 35.3 min (98 %). $^1$H-NMR (60 MHz, $CCl_4$) : δ = 1.25 und 1.45, 2 br s ($CH_2$), 1.97, s(—O—CO—$CH_3$), 2.1-2.4, m, 2.8-3.2, m (—C—H), 3.85, d, J = 7 Hz (—$CH_2$—OAc), 5.61 ppm, s (olefin. H). IR : 1 735 $cm^{-1}$ (Ester-Carbonyl). MS : m/z (%) = 146 (7, $M^+$-36), 131 (5), 117 (6), 105 (10), 91 (28), 80 (100), 78 (27), 67 (27), 63 (7), 51 (3), 43 (32).
$C_{13}H_{18}O_2$ (182.4)

5-Acetoxymethyl-tricyclo [5.2.1.$0^{2,6}$] decan (68) (Darstellung aus 50 analog Beispiel 5)

GC (50 m, FFAP, 230 °C) : $t_r$ = 36.2 min. $^1$H-NMR (60 MHz, $CCl_4$) : δ = 1.3-1.7, m ($CH_2$), 1.97, s (—O—CO—$CH_3$), 2.0-2.3, m (CH), 3.8 ppm, d, J = 7 Hz (—$CH_2$—OAc). IR 1 740 $cm^{-1}$ (Ester). MS : m/z (%) = 148 (23, $M^+$-36), 133 (19), 120 (98), 119 (82), 107 (26), 105 (21), 91 (54), 81 (55), 80 (50), 79 (100), 67 (60), 43 (97). $^{13}$C-NMR : s. Abb. 12.
$C_{13}H_{20}O_2$ (184.4)

5-Hydroxymethyl-tricyclo [5.2.1.$0^{2,6}$] decan (87) (Darstellung aus 48 analog Beispiel 5)

GC (50 m, FFAP, 230 °C) : $t_r$ = 38.6 min. $^1$H-NMR (60 MHz, $CCl_4$) : δ = 1.3-1.7, m ($CH_2$), 1.9-2.3 (CH), 3.6 ppm, d, J = 7 Hz (—$CH_2$OH). IR : 3 350 $cm^{-1}$ (OH). (MS : m/z (%) = 148 (12, $M^+$-18), 135 (85), 133 (22), 120 (65), 119 (61), 107 (63), 105 (19), 93 (58), 91 (64), 81 (65), 80 (67), 79 (90), 67 (100), 55 (19), 41 (45).
$C_{11}H_{18}O$ (166.3)

## Beispiel 16

Parfümol mit Holzcharakter

| | |
|---|---|
| Ylanat (p-tert. Butylcyclohexylacetat) | 200 g |
| Cedernholzöl, Florida | 120 g |
| Galbanum, Resinoid | 50 g |
| Mahagonat® | 70 g |
| Coumarin | 10 g |
| Patchouliöl | 30 g |
| Linalool | 20 g |
| Isobornylacetat | 20 g |
| Linalylacetat | 50 g |
| Labdanum, Resinoid | 20 g |
| Brahmanol® | 50 g |
| Styrollylacetat | 20 g |
| Cyclogalbanat 1 % in Dipropylenglykol | 10 g |

| | |
|---|---|
| Citronellol | 20 g |
| Lactoscaton, 10 % in Dipropylenglykol | 5 g |
| | 700 g |

Diese Grundmischung besitzt einen ausgewogenen holzigen Duft mit Fougère-Aspekten. Durch Zugabe von jeweils 300 g der neuen Verbindungen wurden jeweils neue, originelle Duftaspekte erzielt, und zwar bei Zugabe von

| | Duftcharakteristik |
|---|---|
| a) 11a-d + 12a-d | ausgewogen holzig |
| b) 10a-d + 11a-d | frisch, holzig-krautig |
| c) 13a-d + 11a-d | (Sauge sclarée-Note) holzig, betont würzig-krautige Note |
| d) 22a-d + 11a-d | frisch, holzig, Ambra-Aspekte |
| e) 50 | frisch, holzig, krautig |

Beispiel 17

Parfümöl mit schwer-blumigem Charakter

| | |
|---|---|
| Verbindungen 10a-d + 11a-d (nach Beispiel 4) | 300 g |
| Phenylethylalkohol | 200 g |
| Heptylacetat | 70 g |
| Ylanat (p-tert. Butylcyclohexylacetat) | 80 g |
| Citronellol | 30 g |
| Benzylsalicylat | 40 g |
| Dimethylbenzylcarbinylacetat | 120 g |
| Diisobutylcarbinol | 30 g |
| Eugenol | 10 g |
| Geranylnitril | 60 g |
| Tridecen-2-nitril, 1 %ig in DPG | 10 g |
| Brahmanol® | 40 g |
| | 1 000 g |

Dieses Parfümol mit einem relativ hohen Anteil an den neuen Verbindungen 10a-d + 11a-d zeichnet sich durch eine langanhaftende schwer-blumige, aber auch frische Note aus und ist daher besonders zur Waschmittelparfümierung geeignet.

Beispiel 18

Blumige Komposition

| | |
|---|---|
| Verbindungen 15a-d + 11a-d (nach Beispiel 6) | 300 g |
| Phenylethylalkohol | 100 g |
| Geraniol | 130 g |
| Citronellol | 130 g |
| Phenylessigsäure, 10 %ig in DPG | 10 g |
| α-Jonon | 30 g |
| Linalool | 60 g |
| Geranylacetat | 10 g |
| Phenylethylbutyrat | 5 g |
| Cyclamenaldehyd | 25 g |
| Phenylacetaldehyd, 50 %ig in DMP | 20 g |
| Brahmanol® | 50 g |
| Diheptylacetat | 30 g |
| Indol, 10 %ig in DPG | 10 g |
| Greenylisobutyrat | 50 g |
| Rosenoxid, inaktiv, 10 %ig in DPG | 20 g |
| Isodamascon | 20 g |
| | 1 000 g |

Dieses Parfümöl mit einem hohen Anteil der neuen Verbindungen 15a-d + 11a-d besitzt einen

ausstrahlenden blumigen Duft mit Aspekten von Rosen, Veilchen und Frühlingsblumen.

Beispiel 19

Parfüm-Base mit Holz-Tabak-Ambra-Duft

| | |
|---|---|
| Ethylether 22a-d (+ 11a-d) | 690 g |
| Ambroxan, 10 %ig in DPG | 9 g |
| Lactoscaton, 10 %ig in DPG | 30 g |
| Timberol® | 50 g |
| Ambron® | 30 g |
| Indolal, 10 %ig in DPG | 10 g |
| Patchouliöl | 20 g |
| Mahagonat® | 55 g |
| Oxoisophoron | 5 g |
| Fantesal, 1 %ig in DPG | 1 g |
| | 900 g |

Diese Base mit einem großen Anteil an den neuen Verbindungen 22a-d (+ 11a-d) besitzt einen neuartigen und harmonischen Duftkomplex mit holzigen, krautigen (Tabak) und ambraartigen Aspekten.

**Patentansprüche**

1. Gemische von Tricyclo $[5.2.1.0^{2,6}]$ decan-Derivaten, wobei die Hauptisomeren der allgemeinen Formel A entsprechen, worin

$R^1$ und $R^2$ Wasserstoffatome, bzw. eine Methylgruppe oder ein Wasserstoffatom bedeuten, wobei einer der Substituenten eine Methylgruppe und der andere ein Wasserstoffatom ist,

$R^3$ und $R^4$ Wasserstoffatome, bzw. eine Methylgruppe oder ein Wasserstoffatom bedeuten, wobei einer der Substituenten ein Methylgruppe und der andere ein Wasserstoffatom ist,

Y ein Tetrahydrofuransystem an C-4/C-5 oder eine substituierte Oxymethylgruppe an C-5 und ein Wasserstoffatom an C-4,

$R^5$ ein Wasserstoffatom oder eine $C_1$-$C_6$-Acyl-, $C_1$-$C_6$-Alkyl-, $C_1$-$C_6$-Alkenyl-, $C_1$-$C_4$-Alkoxi-methyl, $C_1$-$C_2$-Alkoxi-carbonyl-, Formylmethyl-, Di-$C_1$-$C_4$-alkoxiethyl-Gruppe

und die gestrichelten Linien eine C-C-Einfachbindung und eine C-C-Doppelbindung oder alternativ zwei C-C-Einfachbindungen bedeuten.

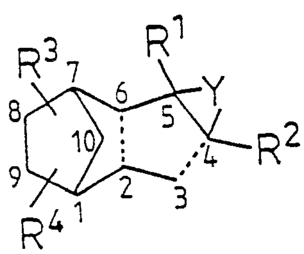

A

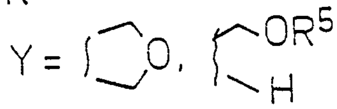

$R^1$, $R^2$, $R^3$, $R^4$ = H
$R^1$, $R^2$ = H, $CH_3$ (1 × $CH_3$)
$R^3$, $R^4$ = H, $CH_3$ (1 × $CH_3$)
$R^5$ = H, $C_1$-$C_6$-Acyl-, $C_1$-$C_6$-Alkyl-, $C_1$-$C_6$-Alkenyl-, $C_1$-$C_4$-Alkoxi-methyl, $C_1$-$C_2$-Alkoxi-carbonyl-Formylmethyl-, Di-$C_1$-$C_4$-alkoxi-ethyl-Gruppe

2. Verfahren zur Herstellung der Gemische der Dimethyl-tricyclo $[5.2.1.0^{2,6}]$ decan-Derivate nach Anspruch 1, dadurch gekennzeichnet, daß man

a) dimeres Methylcyclopentadien selektiv im Norbornanteil unter Verwendung von Nickelborid als Katalysator in polaren, aprotischen, Lösungsmitteln bei einem Wasserstoffdruck von 30 bis 70 bar und einer Temperatur von 20 bis 80 °C hydriert und

b) ggf. die verbleibende Doppelbindung des partiell hydrierten Methylcyclopentadien-Dimeren in an sich bekannter Weise mit Formaldehyd, Paraformaldehyd oder Trioxan in polaren Lösungsmitteln in Gegenwart protischer Säuren oder Lewis-Säuren umsetzt und anschließend

c) ggf. in an sich bekannter Weise Ester, Ether, Acetate oder Oxoaldehyde darstellt.

3. Verfahren nach Anspruch 2, dadurch gekennzeichnet, daß die Umsetzung des partiell hydrierten Methylcyclopentadien-Dimeren mit Paraformaldehyd in Essigsäure/Acetanhydrid unter Verwendung von

starken Säuren, vorzugsweise p-Toluolsulfonsäure, bei erhöhten Temperaturen, vorzugsweise Siedetemperatur, durchgeführt wird.

4. Verfahren nach Anspruch 2, dadurch gekennzeichnet, daß das partiell hydrierte Methylcyclopentadien-Dimere mit einer bei Siedetemperatur hergestellten Lösung von Paraformaldehyd in wasserfreier Ameisensäure bei erhöhter Temperatur, vorzugsweise Siedetemperatur, umgesetzt wird.

5. Verwendung des Gemisches der Dimethyl-tricyclo $[5.2.1.0^{2,6}]$ decan-Derivate gemäß Anspruch 1 als Riechstoffe, bzw. Bestandteil von Riechstoff-Mischungen und Parfümölen zur Parfümierung kosmetischer und technischer Produkte.

6. Verwendung des Gemisches der Dimethyl-tricyclo $[5.2.1.0^{2,6}]$ decan-Derivate gemäß Anspruch 1 als Aromastoff, bzw. Bestandteil von Aroma-Mischungen zur Aromatisierung von Nahrungs- und Genußmitteln.

## Claims

1. Mixtures of tricyclo $[5.2.1.0^{2,6}]$ decane-derivatives, having principal isomers corresponding to the general formula A wherein

$R^1$ and $R^2$ are hydrogen atoms and are a methyl group or a hydrogen atom, respectively, one of the substituents being a methyl group and the other a hydrogen atom,

$R^3$ and $R^4$ are hydrogen atoms and are a methyl group or a hydrogen atom, respectively, one of the substituents being a methyl group and the other a hydrogen atom,

Y is a tetrahydrofurane system at C-4/C-5 or a substituted oxymethyl group at C-5 and a hydrogen atom at C-4,

$R^5$ is a hydrogen atom or a $C_1$-$C_6$-acyl-, $C_1$-$C_6$-alkyl-, $C_1$-$C_6$-alkenyl-, $C_1$-$C_4$-alkoxymethyl, $C_1$-$C_2$alkoxycarbonyl-, formylmethyl-, di-$C_1$-$C_4$-alkoxyethyl group,

and the broken lines indicate a C-C-single bond and a C-C-double bond or alternatively two C-C-single bonds

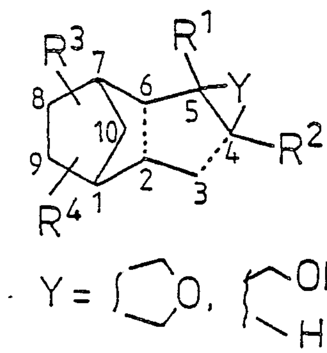

A

$R^1$, $R^2$, $R^3$, $R^4$ = H
$R^1$, $R^2$ = H, $CH_3$ ($1 \times CH_3$)
$R^3$, $R^4$ = H, $CH_3$ ($1 \times CH_3$)
$R^5$ = H, $C_1$-$C_6$-acyl-, $C_1$-$C_6$-alkyl, $C_1$-$C_6$-alkenyl, $C_1$-$C_4$-alkoxymethyl, $C_1$-$C_2$-alkoxycarbonyl-, formylmethyl-, di-$C_1$-$C_4$-alkoxyethyl-group

2. Process for the preparation of mixtures of dimethyltricyclo-$[5.2.1.0^{2,6}]$ decane-derivatives according to claim 1, characterized in that

a) dimeric cyclopentadiene is selectively hydrogenated in the norbornane part using nickel boride as catalyst in polar aprotic solvents at a hydrogen pressure of 30-70 bar and a temperature of 20-80 °C, and

b) if desired the remaining double-bond of the partially hydrogenated methylcyclopentadiene-dimer is reacted with formaldehyde, paraformaldehyde or trioxane in polar solvent in the presence of protic acids or Lewis acids in a manner known per se and thereafter

c) if desired esters, ethers, acetates, or oxoaldehydes are prepared in a manner known per se.

3. Process according to claim 2, characterized in that the reaction of the partially hydrogenated methylcyclopentadiene-dimer with paraformaldehyde is carried out in acetic acid/acetic anhydride using strong acids, preferably p-toluenesulfonic acid, at elevated temperatures, preferably boiling temperature.

4. Process according to claim 2, characterized in that the partially hydrogenated methylcyclopentane-dimer is reacted with a solution prepared at boiling temperature or paraformaldehyde in anhydrous formic acid at an elevated temperature, preferably boiling temperature.

5. Use of the mixture of the dimethyl-tricyclo $[5.2.1.0^{2,6}]$ decane-derivatives according to claim 1 as perfumes and constituent of perfume mixtures and perfume oils for the perfuming of cosmetic and industrial products, respectively.

6. Use of the mixture of the dimethyl-tricyclo $[5.2.1.0^{2,6}]$ decane-derivatives according to claim 1 as

flavoring materials and constituent of flavoring-mixtures for the flavoring of foodstuffs and luxury foodstuffs, respectively.

## Revendications

1. Mélanges de dérivés de tricyclo ($5.2.1.0^{2,6}$) decane, ou les isomères principaux ont la formule générale A

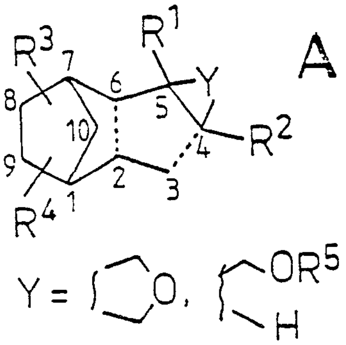

A

$R^1$, $R^2$, $R^3$, $R^4$ = H
$R^1$, $R^2$ = H, $CH_3$ (1 × $CH_3$)
$R^3$, $R^4$ = H, $CH_3$ (1 × $CH_3$)
$R^5$ = H, groupe acyle en $C_1$-$C_6$, alkyle en $C_1$-$C_6$, alcényle en $C_1$-$C_6$ (alcoxy en $C_1$-$C_4$)-méthyle (alcoxy en $C_1$-$C_2$)-carbonyle, formylméthyle, di-(alcoxy en $C_1$-$C_4$)-éthyle.

dans laquelle

$R^1$ et $R^2$ sont des atomes d'hydrogènes, ou encore un groupe méthyle ou un atome d'hydrogène, l'un des substituants étant un groupe méthyle et l'autre un atome d'hydrogène,

$R^3$ et $R^4$ sont des atomes d'hydrogène, ou encore un groupe méthyle ou atome d'hydrogène, l'un des substituants étant un groupe méthyle et l'autre un atome d'hydrogène,
en position C-4/C-5,

Y est un système tétrahydrofuranne, ou en position C-5 un groupe oxyméthyle substitué et en position C-4 un atome d'hydrogène,

$R^5$ est un atome d'hydrogène ou un groupe acyle en $C_1$-$C_6$, alkyle en $C_1$-$C_6$, alcényle en $C_1$-$C_6$, (alcoxy en $C_1$-$C_4$)-méthyle, (alcoxy en $C_1$-$C_2$)-carbonyle, formylméthyle, di(alcoxy en $C_1$-$C_4$)-éthyle,
et les lignes en tirets représentent une liaison simple C-C ou une double liaison C-C ou encore deux liaisons simples C-C.

2. Procédé pour la préparation de mélanges de dérivés de diméthyl-tricyclo ($5.2.1.0^{2,6}$) décane selon la revendication 1, caractérisé

a) en ce qu'on hydrogène sélectivement du méthylcyclopentadiène dimère dans la partie norbornane, en utilisant du borure de nickel comme catalyseur, dans des solvants aprotiques polaires, sous une pression d'hydrogène de 30 à 70 bar et à une température de 20 à 80 °C.

b) qu'on fait éventuellement réagir la double liaison restante des dimères partiellement hydrogénés du cyclopentadiène, d'une manière connue en soi, à l'aide de formaldéhyde, de paraformaldéhyde ou de trioxanne, dans des solvants polaires en présence d'acides protiques ou d'acides de Lewis, puis

c) qu'on prépare éventuellement, d'une manière connue en soi, des esters, des éthers, des acétates ou des oxoaldéhydes.

3. Procédé selon la revendication 2, caractérisé en ce que la réaction des dimères partiellement hydrogénés de méthylcyclopentadiène avec le paraformaldéhyde est réalisée dans de l'acide acétique/anhydride acétique, en utilisant des acides forts, de préférence de l'acide p-toluènesulfonique, à hautes températures, de préférence à la température d'ébullition.

4. Procédé selon la revendication 2, caractérisé en ce que les dimères partiellement hydrogénés du méthylcyclopentadiène sont mis à réagir, à une température élevée, de préférence à la température d'ébullition, avec une solution, préparée à la température d'ébullition, de paraformaldéhyde dans de l'acide formique anhydre.

5. Utilisation du mélange de dérivés de diméthyl-tricyclo-($5.2.1.0^{2,6}$) décane selon la revendication 1 comme produits odorants, ou comme constituants de mélanges de produits odorants et de parfums, pour le parfumage de produits cosmétiques et techniques.

6. Utilisation du mélange des dérivés de diméthyltricyclo ($5.2.1.0^{2,6}$) décane selon la revendication 1 comme substance aromatique, ou constituant de mélanges aromatiques destinés à aromatiser les produits alimentaires et produits connexes.

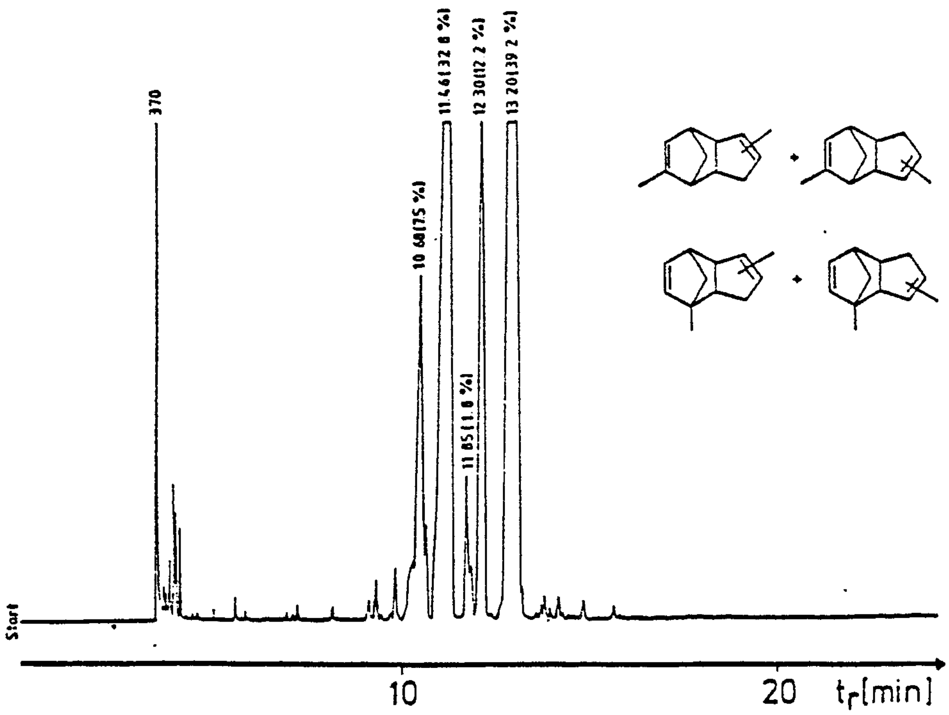


Abb. 1: Gaschromatogramm [50 m, Glaskapillare FFAP, 40-250°C, 8°C/min] von dimerem Methylcyclopentadien

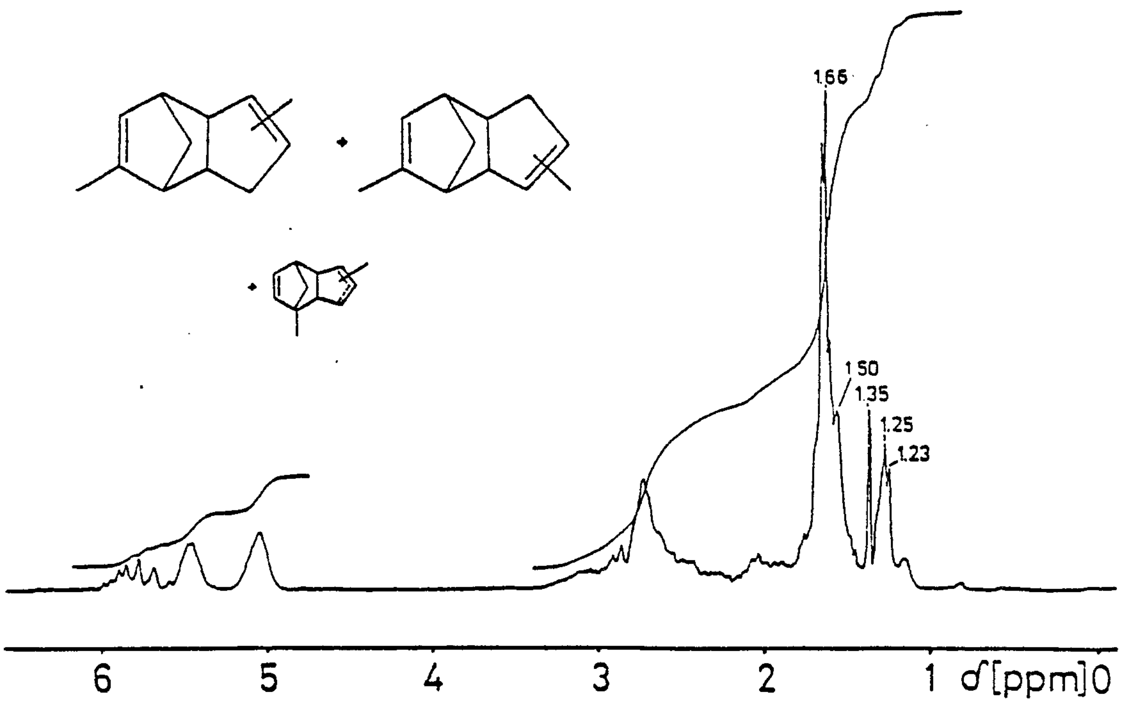


Abb. 2: $^{1}$H-NMR-Spektrum [$CCl_4$, 60 MHz] von dimerem Methylcyclopentadien

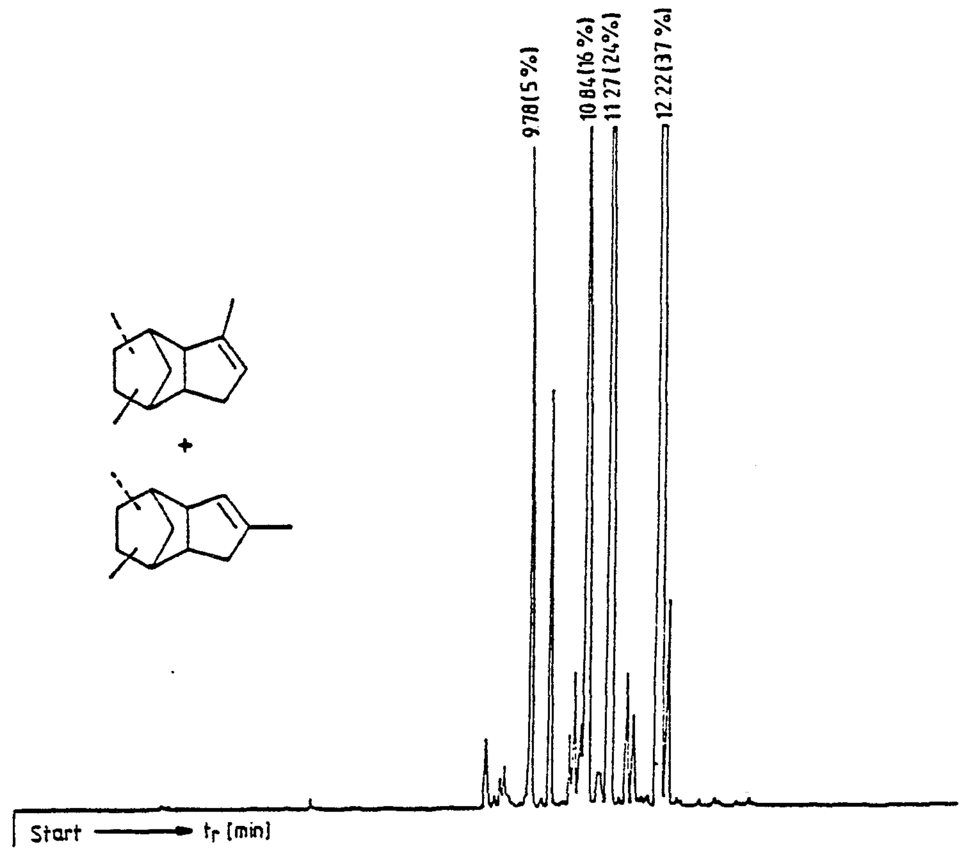


Abb. 3: Gaschromatogramm [50 m, Glaskapillare FFAP, 40-250°C, 8°C/min] von 8a-f

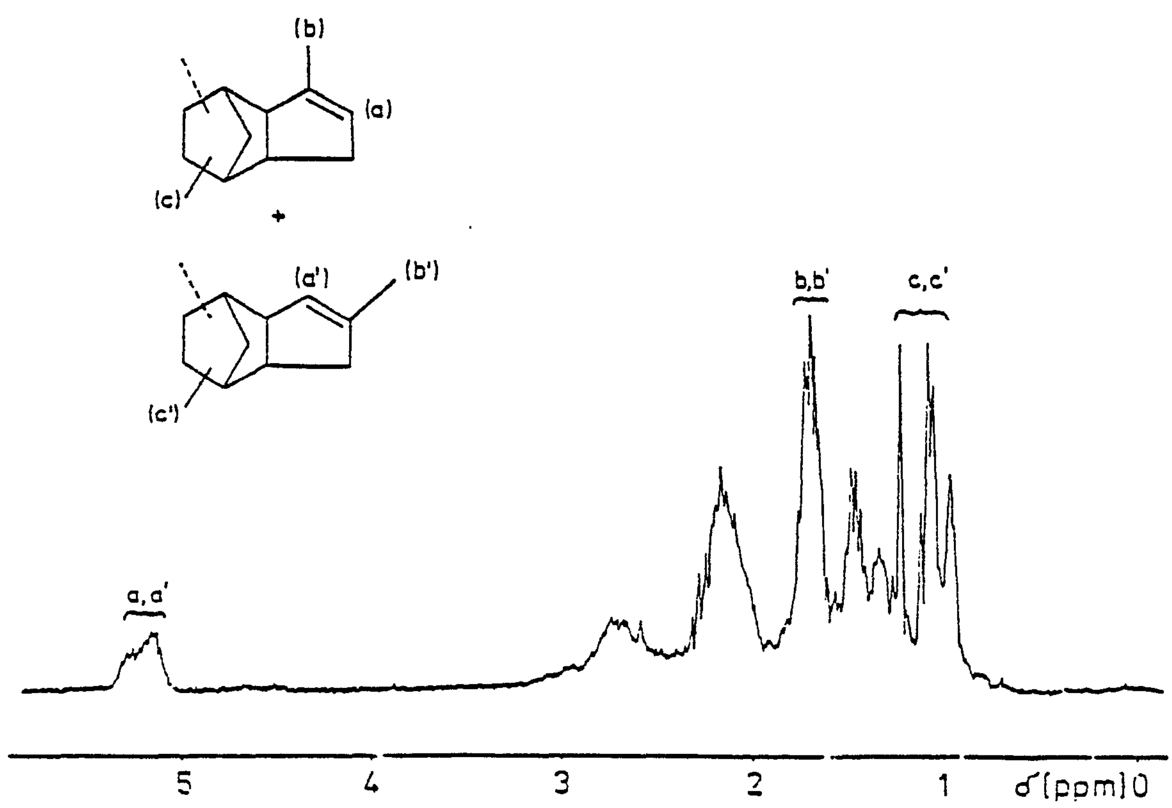


Abb. 4: $^{1}$H-NMR-Spektrum [$CCl_4$, 60 MHz] von 8a-f

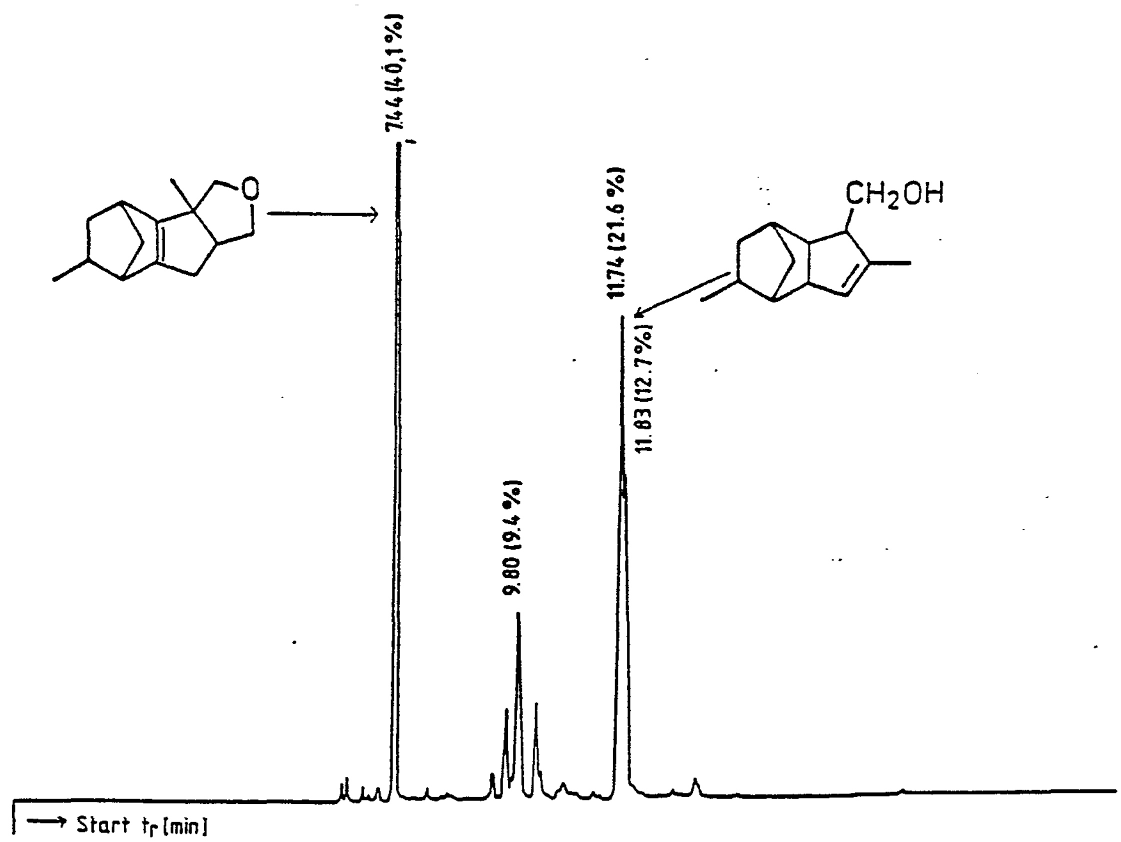


Abb. 5: Gaschromatogramm des Produktgemisches nach Beispiel 3

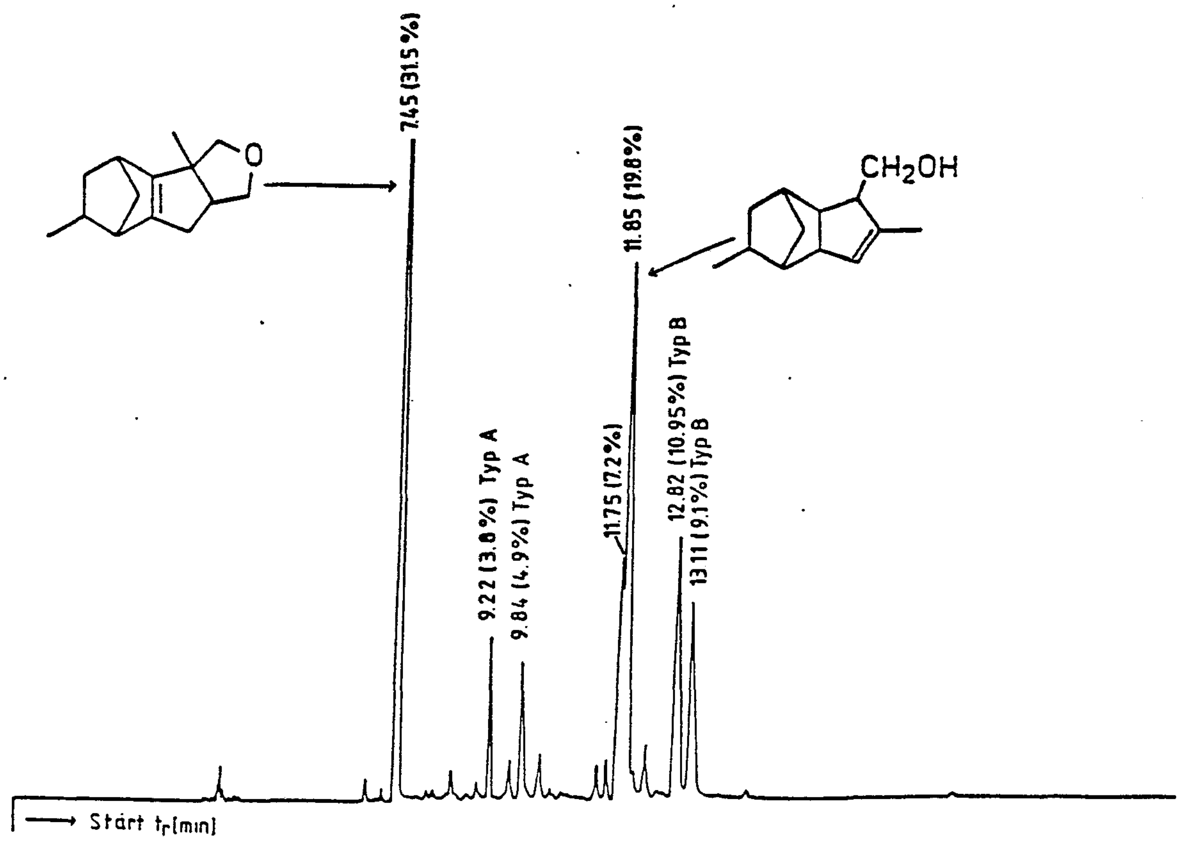


Abb. 6: Gaschromatogramm des Produktgemisches nach Beispiel 4

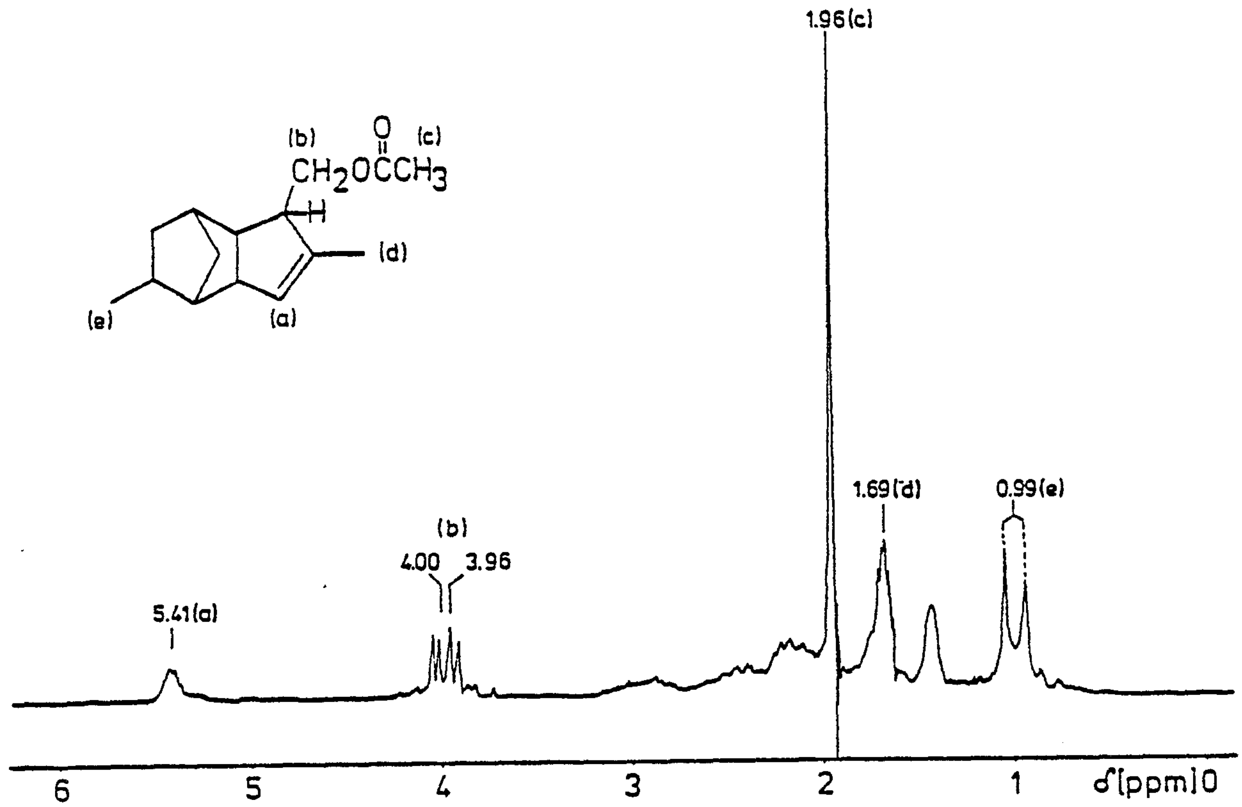


Abb. 7: $^1$H-NMR-Spektrum ($CCl_4$, 60 MHz) von 10d

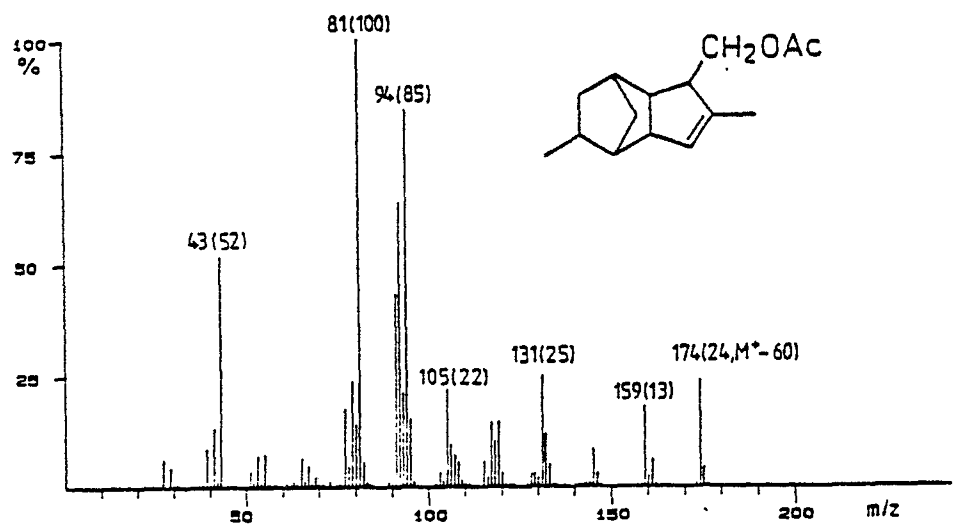


Abb. 8: Massenspektrum von 10d

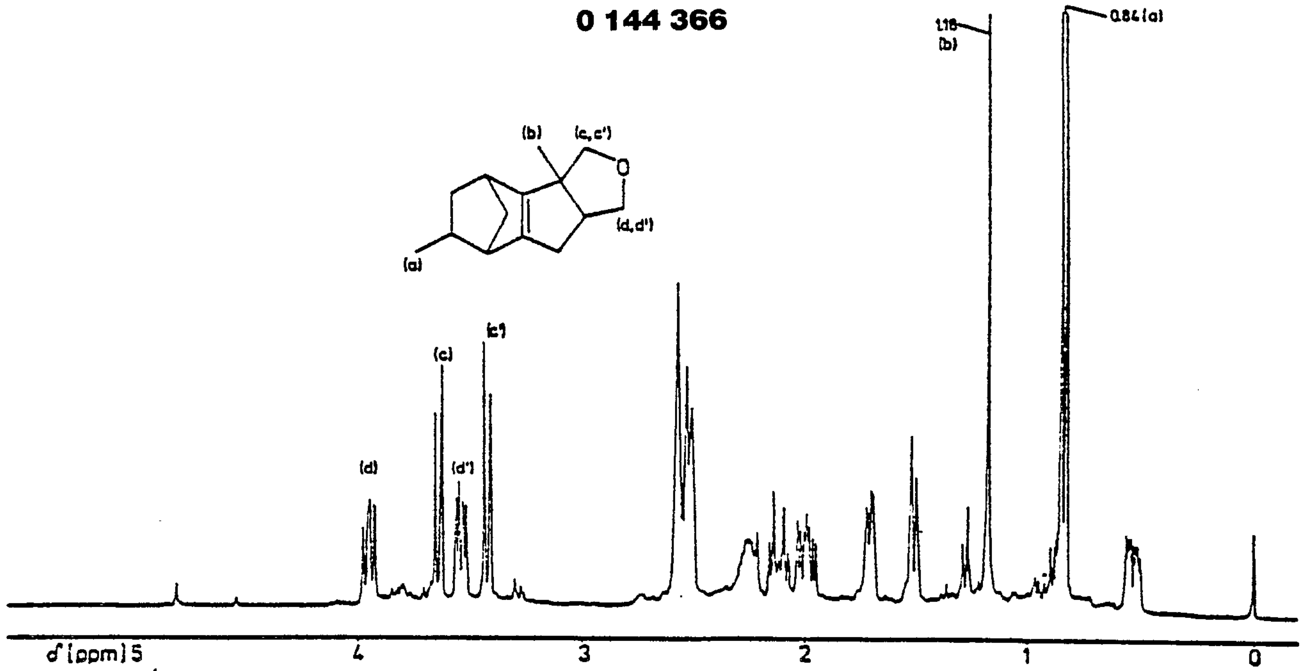


Abb. 9: $^{1}$H-NMR-Spektrum (270 MHz, $CDCl_3$) von 11d

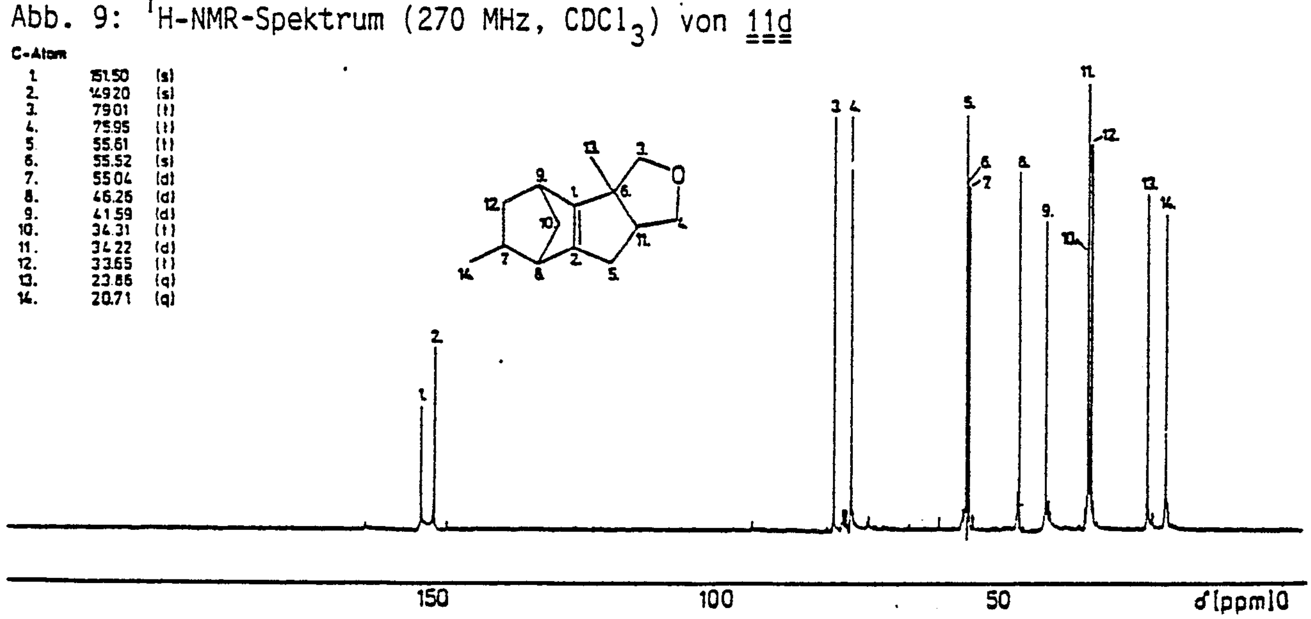


| C-Atom | | |
|---|---|---|
| 1. | 151.50 | (s) |
| 2. | 149.20 | (s) |
| 3. | 79.01 | (t) |
| 4. | 75.95 | (t) |
| 5. | 55.61 | (t) |
| 6. | 55.52 | (s) |
| 7. | 55.04 | (d) |
| 8. | 46.26 | (d) |
| 9. | 41.59 | (d) |
| 10. | 34.31 | (t) |
| 11. | 34.22 | (d) |
| 12. | 33.65 | (t) |
| 13. | 23.86 | (q) |
| 14. | 20.71 | (q) |

Abb. 10: $^{13}$C-NMR-Spektrum von 11d

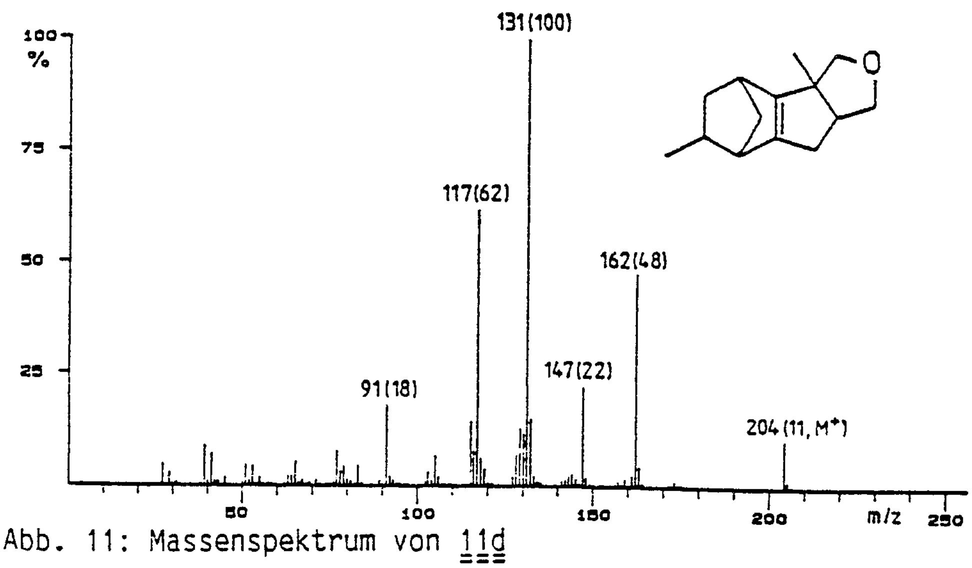


Abb. 11: Massenspektrum von 11d

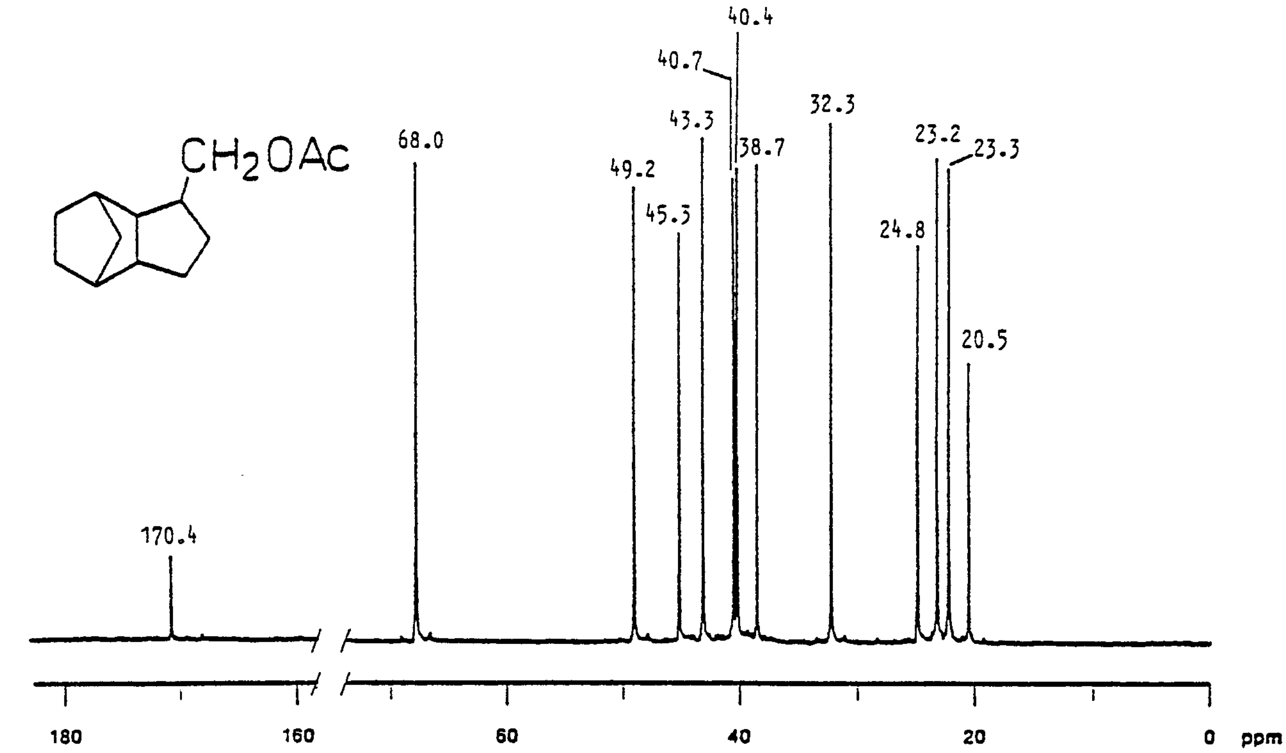


Abb. 12: $^{13}C$-NMR-Spektrum ($CDCl_3$) von 68

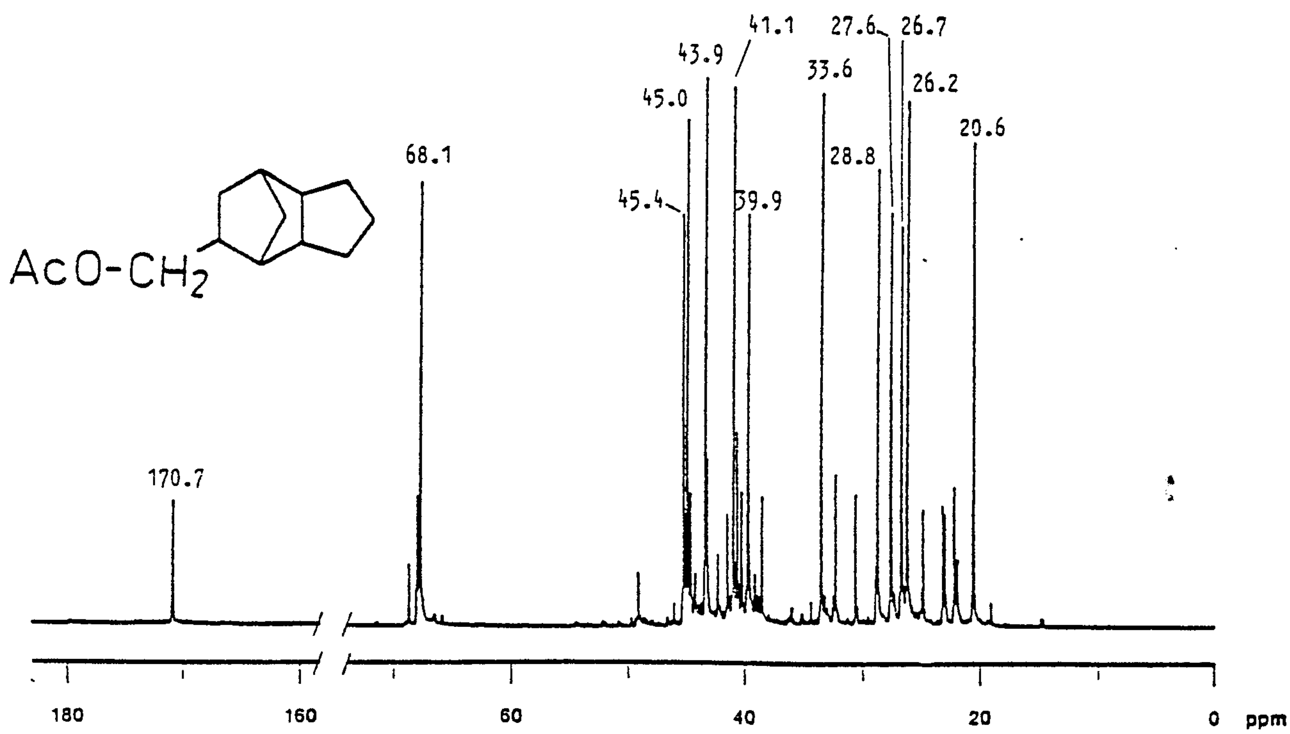


Abb. 13: $^{13}C$-NMR-Spektrum ($CDCl_3$) von C (R = Acetyl, Isomerengemisch)

- Darstellung analog DOS 26 54 268 -